(19) Europäisches Patentamt
European Patent Office
Office européen des brevets

(11) EP 2 445 860 B1

(12) EUROPÄISCHE PATENTSCHRIFT

(45) Veröffentlichungstag und Bekanntmachung des Hinweises auf die Patenterteilung:
**29.05.2013 Patentblatt 2013/22**

(21) Anmeldenummer: **10725137.3**

(22) Anmeldetag: **11.06.2010**

(51) Int Cl.:
***C07C 51/00*** *(2006.01)* ***C07C 51/44*** *(2006.01)*
***C07C 53/02*** *(2006.01)*

(86) Internationale Anmeldenummer:
**PCT/EP2010/058208**

(87) Internationale Veröffentlichungsnummer:
**WO 2010/149507 (29.12.2010 Gazette 2010/52)**

# (54) VERFAHREN ZUR HERSTELLUNG VON AMEISENSÄURE

METHOD FOR PRODUCING FORMIC ACID

PROCÉDÉ DE PRÉPARATION D'ACIDE FORMIQUE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **26.06.2009 EP 09008399**
**24.03.2010 EP 10157452**

(43) Veröffentlichungstag der Anmeldung:
**02.05.2012 Patentblatt 2012/18**

(73) Patentinhaber: **BASF SE**
**67056 Ludwigshafen (DE)**

(72) Erfinder:
- **SCHAUB, Thomas**
**67433 Neustadt (DE)**
- **PACIELLO, Rocco**
**67098 Bad Dürkheim (DE)**
- **MOHL, Klaus-Dieter**
**68766 Hockenheim (DE)**
- **SCHNEIDER, Daniel**
**68159 Mannheim (DE)**
- **SCHÄFER, Martin**
**67269 Grünstadt (DE)**
- **RITTINGER, Stefan**
**68199 Mannheim (DE)**

(56) Entgegenhaltungen:
**EP-A1- 0 181 078**
**EP-A1- 0 583 695**
**EP-A2- 0 095 321**
**WO-A1-2006/021411**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Herstellung von Ameisensäure durch Hydrierung von Kohlendioxid in Gegenwart eines homogenen Katalysators enthaltend ein Element aus der 8., 9. oder 10. Gruppe des Periodensystems, eines tertiären Amins (I) und eines polaren Lösungsmittels (III) bei einem Druck von 0,2 bis 30 MPa abs und einer Temperatur von 20 bis 200°C unter Ausbildung zweier Flüssigphasen, Trennung der einen, mit dem Ameisensäure/Amin-Addukt (II) angereicherten Flüssigphase (A) von der anderen Flüssigphase (B) und Rückführung der Flüssigphase (B) zum Hydrierreaktor.

Addukte aus Ameisensäure und tertiären Aminen können thermisch in freie Ameisensäure und tertiäres Amin gespalten werden und dienen daher als Intermediat in der Herstellung von Ameisensäure. Ameisensäure ist ein wichtiges und vielseitig einsetzbares Produkt. Sie wird beispielsweise zum Ansäuern bei der Herstellung von Futtermitteln, als Konservierungsmittel, als Desinfektionsmittel, als Hilfsstoff in der Textil- und Lederindustrie, als Gemisch mit ihren Salzen zur Enteisung von Flugzeugen und Start- und Landebahnen sowie als Synthesebaustein in der chemischen Industrie verwendet.

Die genannten Addukte aus Ameisensäure und tertiären Aminen können auf verschiedene Art und Weise hergestellt werden, beispielsweise (i) durch direkte Umsetzung des tertiären Amins mit Ameisensäure, (ii) durch Hydrolyse von Methylformiat zu Ameisensäure in Gegenwart des tertiären Amins oder mit nachgeschalteter Extraktion des Hydrolyseaustrags mit dem tertiären Amin, oder (iii) durch katalytische Hydratisierung von Kohlenmonoxid oder Hydrierung von Kohlendioxid zu Ameisensäure in Gegenwart des tertiären Amins. Das letztgenannte Verfahren der katalytischen Hydrierung von Kohlendioxid hat den besonderen Charme, dass Kohlendioxid in großen Mengen verfügbar und hinsichtlich seiner Quelle flexibel ist.

WO 2008/116,799 offenbart ein Verfahren zur Hydrierung von Kohlendioxid in Gegenwart eines in Lösung suspendierten oder homogen gelösten Katalysators enthaltend ein Übergangsmetall der VIII. Nebengruppe (Gruppe 8, 9, 10), eines tertiären Amins mit mindestens einer Hydroxylgruppe und eines polaren Lösungsmittels zu einem Addukt aus Ameisensäure und dem tertiären Amin. Durch die Hydroxylgruppe(n) im tertiären Amin wird gegenüber dem sonst üblicherweise eingesetzten Triethylamin eine erhöhte Kohlendioxid-Löslichkeit erreicht. Als bevorzugte Homogenkatalysatoren sind $RuH_2L_4$ mit einzähnigen Phosphor-basierten Liganden L und $RuH_2(LL)_2$ mit zweizähnigen Phosphor-basierten Liganden LL und als besonders bevorzugt $RuH_2[P(C_6H_5)_3]_4$ genannt. Als polare Lösungsmittel sind Alkohole, Ether, Sulfolane, Dimethylsulfoxid und Amide, deren Siedepunkt bei Normaldruck mindestens 5°C oberhalb dem von Ameisensäure liegt, genannt. Auch die bevorzugt einzusetzenden tertiären Amine weisen einen Siedepunkt oberhalb dem der Ameisensäure auf. Da keine Phasentrennung stattfindet, erfolgt die Aufarbeitung des gesamten Reaktionsaustrags durch Destillation, gegebenenfalls nach vorheriger Abtrennung des Katalysators, wobei dabei das gebildete Addukt aus Ameisensäure und dem tertiären Amin thermisch gespalten und die freigesetzte Ameisensäure als Kopfprodukt gewonnen wird. Das Sumpfprodukt enthaltend tertiäres Amin, polares Lösungsmittel und gegebenenfalls Katalysator wird zur Hydrierstufe zurückgeführt.

Nachteilig an diesem Verfahren ist die Zuführung des gesamten flüssigen Reaktionsaustrags in die Vorrichtung zur thermischen Spaltung und Destillation, gegebenenfalls nach vorgeschalteter spezifischer Abtrennung des homogenen Katalysators durch einen separaten Verfahrensschritt wie beispielsweise einer Extraktions-, Adsorptions-oder Ultrafiltrationsstufe. Als Folge davon ist die Vorrichtung zur thermischen Spaltung und Destillation hinsichtlich der höheren Flüssigkeitsbelastung als auch hinsichtlich der spezifischeren Trenneigenschaften größer und komplexer auszulegen, was sich unter anderem auch in den Investitionskosten niederschlägt (zum Beispiel via Ingenieurleistung, Material, Flächenbedarf). Zudem bedingt die höhere Flüssigkeitsbelastung auch einen höheren Energiebedarf.

Die grundlegenden Arbeiten zur katalytische Hydrierung von Kohlendioxid zu Ameisensäure wurden jedoch bereits in den 1970er und 1980er Jahren gemacht. Als Ausfluss davon dürften wohl auch die in EP 0 095 321 A, EP 0 151 510 A und EP 0 181 078 A zum Patent angemeldeten Verfahren der BP Chemicals Ltd. zu verstehen sein. Alle drei Schriften beschreiben die Hydrierung von Kohlendioxid in Gegenwart eines homogenen Katalysators enthaltend ein Übergangsmetall der VIII. Nebengruppe (Gruppe 8, 9, 10), eines tertiären Amins und eines polaren Lösungsmittels zu einem Addukte aus Ameisensäure und dem tertiären Amin. Als bevorzugte Homogenkatalysatoren sind in EP 0 095 321 A und EP 0 181 078 A jeweils Rutheniumbasierte Carbonyl-, Halogenid- und/oder Triphenylphosphin-haltige Komplexkatalysatoren und in EP 0 151 510 A Rhodium/Phosphin-Komplexe genannt. Bevorzugte tertiäre Amine sind $C_1$-$C_{10}$-Trialkylamine, insbesondere die kurzkettigen $C_1$-$C_4$-Trialkylamine, sowie cyclische und/oder verbrückte Amine wie 1,8-Diazabicyclo[5.4.0]undec-7-en, 1,4-Diazabicyclo[2.2.2]octan, Pyridin oder Picoline. Die Hydrierung erfolgt bei einem Kohlendioxid-Partialdruck von bis zu 6 MPa (60 bar), einem Wasserstoff-Partialdruck von bis zu 25 MPa (250 bar) und einer Temperatur von etwa Raumtemperatur bis 200°C.

EP 0 095 321 A und EP 0 151 510 A lehren den Einsatz eines Alkohols als polares Lösungsmittel. Da jedoch primäre Alkohole zur Bildung von Ameisensäureestern (organischen Formiaten) neigen, sind sekundäre Alkohole, insbesondere Isopropanol, bevorzugt. Zudem wird die Gegenwart von Wasser als vorteilhaft beschrieben. Gemäß den Beispielen aus EP 0 095 321 A erfolgt die Aufarbeitung des Reaktionsaustrags durch eine direkt nachfolgende zweistufige

Destillation, bei der in der ersten Stufe die Leichtsieder Alkohol, Wasser, tertiäres Amin und in der zweiten Stufe unter Vakuumbedingungen das Addukt aus Ameisensäure und dem tertiären Amin über Kopf abgetrennt werden. EP 0 151 510 A lehrt ebenfalls eine destillative Aufarbeitung, jedoch unter Verweis auf EP 0 126 524 A mit nachfolgendem Austausch des tertiären Amins im destillativ abgetrennten Addukt vor dessen thermischer Spaltung durch eine schwächere, weniger flüchtige Stickstoffbase, um die nachfolgende thermische Spaltung zur Darstellung der freien Ameisensäure zu erleichtern beziehungsweise erst möglich zu machen.

EP 0 181 078 A lehrt die gezielte Auswahl des polaren Lösungsmittels anhand von drei wesentlichen Kriterien, die gleichzeitig erfüllt sein müssen:

(i) der homogene Katalysator muss im polaren Lösungsmittel löslich sein;
(ii) das polare Lösungsmittel darf die Hydrierung nicht nachteilig beeinflussen; und
(iii) das gebildete Addukt aus Ameisensäure und dem tertiären Amin sollte leicht vom polaren Lösungsmittel abtrennbar sein.

Als besonders geeignete polare Lösungsmittel sind verschiedene Glykole und Phenylpropanole genannt.

Die Aufarbeitung des Reaktionsaustrags gemäß der Lehre von EP 0 181 078 A erfolgt derart, dass zunächst in einem Verdampfer die gasförmigen Komponenten (vor allem nicht umgesetzte Edukte Wasserstoff und Kohlendioxid) über Kopf und der homogene Katalysator gelöst im polaren Lösungsmittel über Sumpf abgetrennt und zur Hydrierstufe zurückgeführt werden. Aus der verbleibenden Flüssigphase enthaltend das Addukt aus Ameisensäure und dem tertiären Amin, freies tertiäres Amin und gegebenenfalls Wasser wird dann anschließend das Addukt aus Ameisensäure und dem tertiären Amin abgetrennt und der verbleibende Teil der Flüssigphase enthaltend das freie tertiäre Amin und gegebenenfalls Wasser zur Hydrierstufe zurückgeführt. Die Abtrennung kann durch Destillation oder durch Phasentrennung des Zweiphasensystems (Dekantierung) erfolgen.

Eine weitere wesentliche Lehre aus EP 0 181 078 A ist der anschließende, zwingende Austausch des tertiären Amins im abgetrennten Addukt vor dessen thermischer Spaltung durch eine schwächere, weniger flüchtige Stickstoffbase, um die nachfolgende thermische Spaltung zur Darstellung der freien Ameisensäure zu erleichtern beziehungsweise erst möglich zu machen. Als besonders geeignete schwächere Stickstoffbasen sind Imidazolderivate wie beispielsweise 1-n-Butylimidazol genannt.

Nachteilig am Verfahren des EP 0 181 078 A ist die sehr aufwändige, vierstufige Aufarbeitung des Reaktionsaustrags durch

(i) Abtrennung der gasförmigen Komponenten sowie des homogenen Katalysators und des polaren Lösungsmittels in einem Verdampfer und Rückführung zur Hydrierstufe;
(ii) Abtrennung des Addukts aus Ameisensäure und dem tertiären Amin in einer Destillationskolonne oder einem Phasenscheider und Rückführung des verbleibenden Flüssigkeitsstroms zur Hydrierstufe;
(iii) Austausch des tertiären Amins im Addukt aus Ameisensäure und dem tertiären Amin durch eine schwächere, weniger flüchtige Stickstoffbase in einem Reaktionskessel mit aufgesetzter Destillationskolonne und Rückführung des freigesetzten tertiären Amins zur Hydrierstufe; und
(iv) thermische Spaltung des Addukts aus Ameisensäure und der schwächeren Stickstoffbase und Rückführung der freigesetzten schwächeren Stickstoffbase zur Basenaustauschstufe.

Ein weiterer, wesentlicher Nachteil am Verfahren des EP 0 181 078 A, sowie auch an den Verfahren der EP 0 095 321 A und EP 0 151 510 A ist die Tatsache, dass sich das Addukt aus Ameisensäure und dem tertiären Amin in Gegenwart des homogenen Katalysators bei der Aufarbeitung im Verdampfer zum Teil wieder in Kohlendioxid und Wasserstoff rückspaltet. In EP 0 329 337 A ist daher als Lösung des Problems die Zugabe eines Zersetzungsinhibitors vorgeschlagen, welcher den homogenen Katalysator reversibel inhibiert. Als bevorzugte Zersetzungsinhibitoren sind Kohlenmonoxid und Oxidationsmittel genannt. Nachteilig daran ist jedoch die Einführung weiterer Substanzen in das Gesamtverfahren und die Notwendigkeit, den inhibierten homogenen Katalysator vor dessen erneutem Einsatz wieder zu aktivieren.

Auch EP 0 357 243 A befasst sich mit dem Nachteil der teilweisen Rückspaltung des Addukts aus Ameisensäure und dem tertiären Amin im Verfahren des EP 0 181 078 A durch die gemeinsame Aufarbeitung des Reaktionsaustrags im Verdampfer. Das in EP 0 357 243 A vorgeschlagene Verfahren lehrt bei der katalytischen Hydrierung des Kohlendioxids zu einem Addukt aus Ameisensäure und tertiärem Amin den Einsatz eines homogenen Katalysators enthaltend ein Übergangsmetall der VIII. Nebengruppe (Gruppe 8, 9, 10), eines tertiären Amins und zweier unterschiedlicher Lösungsmittel, nämlich eines unpolaren und eines polaren, jeweils inerten Lösungsmittels, welche zwei nicht-mischbare flüssige Phasen bilden. Als unpolare Lösungsmittel sind aliphatische und aromatische Kohlenwasserstoffe, jedoch auch Phosphine mit aliphatischen und/oder aromatischen Kohlenwasserstoffresten genannt. Als polare Lösungsmittel sind Wasser, Glycerin, Alkohole, Polyole, Sulfolane oder deren Mischungen genannt, wobei Wasser bevorzugt ist. Im unpolaren Lösungsmittel löst sich der homogene Katalysator, im polaren Lösungsmittel das Addukt aus Ameisensäure und tertiärem Amin. Nach Beendigung der Reaktion werden beide Flüssigphasen separiert, beispielsweise durch Dekantierung, und die unpolare Phase enthaltend den homogenen Katalysator und das unpolare Lösungsmittel zur Hydrierstufe zurückgeführt. Die polare Phase enthaltend das Addukt aus Ameisensäure und tertiärem Amin und das polare Lösungsmittel wird dann einem zwingenden Austausch des tertiären Amins im Addukt vor dessen thermischer Spaltung durch eine schwächere, weniger flüchtige Stickstoffbase unterworfen, um die nachfolgende thermische Spaltung zur Darstellung der freien Ameisensäure zu erleichtern beziehungsweise erst möglich zu machen. Analog EP 0 181 078 A sind auch hier Imidazolderivate wie beispielsweise 1-n-Butylimidazol als besonders geeignete schwächere Stickstoffbasen genannt.

Nachteilig am Verfahren des EP 0 357 243 A ist die sehr aufwändige, dreistufige Aufarbeitung des Reaktionsaustrags durch

(i) Trennung der beiden Flüssigphasen und Rückführung der den homogenen Katalysator und das unpolare Lösungsmittel enthaltenden Phase zur Hydrierstufe;
(ii) Austausch des tertiären Amins im Addukt aus Ameisensäure und dem tertiären Amin der anderen Phase durch eine schwächere, weniger flüchtige Stickstoffbase in einem Reaktionskessel mit aufgesetzter Destillationskolonne und Rückführung des freigesetzten tertiären Amins zur Hydrierstufe; und
(iii) thermische Spaltung des Addukts aus Ameisensäure und der schwächeren Stickstoffbase und Rückführung der freigesetzten schwächeren Stickstoffbase zur Basenaustauschstufe.

Ein weiterer Nachteil am Verfahren des EP 0 357 243 A ist der Einsatz zweier Lösungsmittel und somit die Einführung einer weiteren Substanz in das Gesamtverfahren.

Alternativ offenbart EP 0 357 243 A auch die Möglichkeit, nur ein Lösungsmittel einzusetzen. In diesem Fall entfällt die Zugabe des polaren Lösungsmittels, in dem sich ansonsten das Addukt aus Ameisensäure und dem tertiären Amin lösen würde. Das einzige hierbei eingesetzte Lösungsmittel ist das unpolare Lösungsmittel, welches den homogenen Katalysator löst. Auch bei dieser Alternative ist jedoch die sehr aufwändige, dreistufige Aufarbeitung wie oben beschrieben von Nachteil.

DE 44 31 233 A beschreibt ebenfalls die Hydrierung von Kohlendioxid in Gegenwart eines Katalysators enthaltend ein Übergangsmetall der VIII. Nebengruppe (Gruppe 8, 9, 10), eines tertiären Amins und eines polaren Lösungsmittels und Wasser zu einem Addukt aus Ameisensäure und dem tertiären Amin, bei dem jedoch der Katalysator heterogen vorliegt und die Aktivkomponente auf einem inerten Träger aufgebracht ist. Bevorzugte tertiäre Amine sind $C_1$-$C_8$-Trialkylamine, Polyamine mit 2 bis 5 Aminogruppen, aromatische Stickstoffheterocyclen wie Pyridin oder N-Methylimidazol, sowie cyclische und/oder verbrückte Amine wie N-Methylpiperidin, 1,8-Diazabicyclo[5.4.0]undec-7-en oder 1,4-Diazabicyclo[2.2.2]octan. Als geeignete polare Lösungsmittel sind die niedrig siedenden $C_1$-$C_4$-Monoalkohole genannt, wobei analog EP 0 095 321 A sekundäre Alkohole bevorzugt sind. Die Hydrierung erfolgt bei einem Gesamtdruck von 4 bis 20 MPa (40 bis 200 bar) und einer Temperatur von 50 bis 200°C. Zur Aufarbeitung des gebildeten Addukts aus Ameisensäure und tertiärem Amin lehrt DE 44 31 233 A den Einsatz bekannter Verfahren unter explizitem Verweis auf die in EP 0 357 243 A offenbarte Aufarbeitung unter Austausch des tertiären Amins im Addukt aus Ameisensäure und dem tertiären Amin durch eine schwächere, weniger flüchtige Stickstoffbase. Analog dem Verfahren nach EP 0 357 243 A ist auch beim Verfahren nach DE 44 31 233 A die sehr aufwändige, dreistufige Aufarbeitung des Reaktionsaustrags nachteilig.

Aufgabe der vorliegenden Erfindung war es, ein Verfahren zur Herstellung von Ameisensäure durch Hydrierung von Kohlendioxid zu finden, welches die genannten Nachteile des Standes der Technik nicht oder nur in deutlich vermindertem Ausmaß aufweist und welches konzentrierte Ameisensäure in hoher Ausbeute und hoher Reinheit zu gewinnen vermag. Ferner soll das Verfahren eine einfache oder zumindest einfachere Verfahrensführung erlauben als im Stand der Technik beschrieben, wie beispielsweise durch ein anderes, einfacheres Verfahrenskonzept, einfachere Verfahrensstufen, eine geringere Anzahl an Verfahrensstufen oder einfachere Apparate. Des Weiteren soll das Verfahren auch möglichst energiegünstig durchgeführt werden können.

Demgemäß wurde ein Verfahren zur Herstellung von Ameisensäure durch Hydrierung von Kohlendioxid in Gegenwart eines Katalysators enthaltend ein Element aus der 8., 9. oder 10. Gruppe des Periodensystems, eines tertiären Amins (I) und eines polaren Lösungsmittels (III) bei einem Druck von 0,2 bis 30 MPa abs und einer Temperatur von 20 bis 200°C unter Ausbildung zweier Flüssigphasen, Trennung der einen, mit dem Ameisensäure/Amin-Addukt (II) angereicherten Flüssigphase (A) von der anderen Flüssigphase (B) und Rückführung der Flüssigphase (B) zum Hydrierreaktor gefunden, das dadurch gekennzeichnet ist, dass man als Katalysator einen homogenen Katalysator einsetzt und dieser zusammen mit dem tertiären Amin (I) in der Flüssigphase (B) angereichert vorliegt

(a) als tertiäres Amin (I) ein Amin einsetzt, welches bei einem Druck von 1013 hPa abs einen um mindestens 5°C höheren Siedepunkt als Ameisensäure aufweist und in der Flüssigphase (B) angereichert vorliegt;

(b) als polares Lösungsmittel (III) ein Lösungsmittel einsetzt, dessen elektrostatischer Faktor $\geq 200 \cdot 10^{-30}$ Cm beträgt und welches bei einem Druck von 1013 hPa abs einen um mindestens 5°C höheren Siedepunkt als Ameisensäure aufweist und in der Flüssigphase (A) angereichert vorliegt;

(c) das Ameisensäure/Amin-Addukt (II) der abgetrennten Flüssigphase (A) in einer Destillationseinheit thermisch in freie Ameisensäure und freies tertiäres Amin (I) spaltet;

(d) die freie Ameisensäure destillativ entfernt; und

(e) das im Sumpf der Destillationseinheit enthaltene freie tertiäre Amin (I) und das polare Lösungsmittel (III) zum Hydrierreaktor rückführt.

Der beim erfindungsgemäßen Verfahren bei der Hydrierung von Kohlendioxid einzusetzende homogene Katalysator enthält ein Element aus der 8., 9. oder 10. Gruppe des Periodensystems, also Fe, Co, Ni, Ru, Rh, Pd, Os, Ir und/oder Pt. Bevorzugt enthält der Katalysator Ru, Rh, Pd, Os, Ir und/oder Pt, besonders bevorzugt Ru, Rh und/oder Pd, und ganz besonders bevorzugt Ru. Die katalytisch aktiven Komponenten können die Metalle selbst, beispielsweise in fein verteilter Form, oder auch Komplexverbindungen sein.

Die genannten Elemente liegen in Form von komplexartigen Verbindungen im Reaktionsgemisch homogen gelöst vor. Der homogene Katalysator ist dabei so auszuwählen, dass dieser zusammen mit dem tertiären Amin (I) in der Flüssigphase (B) angereichert vorliegt. Unter "angereichert" ist dabei ein Verteilungskoeffizient des homogenen Katalysators

$$P = [\text{Konzentration homogener Katalysator in Flüssigphase (B)}] / [\text{Konzentration homogener Katalysator in Flüssigphase (A)}]$$

von > 1 zu verstehen. Bevorzugt liegt der Verteilungskoeffizient bei ≥ 10 und besonders bevorzugt brei ≥ 20. Die Auswahl des homogenen Katalysators erfolgt im Allgemeinen durch einen einfachen Versuch, in dem der Verteilungskoeffizient des gewünschten homogenen Katalysators unter den geplanten Verfahrensbedingungen experimentell bestimmt wird.

Wegen ihrer guten Löslichkeit in tertiären Aminen (I) verwendet man beim erfindungsgemäßen Verfahren als homogene Katalysatoren bevorzugt einen metallorganischen Komplex, welcher ein Element aus der 8., 9. oder 10. Gruppe des Periodensystems und mindestens eine Phosphin-Gruppe mit mindestens einem unverzweigten oder verzweigten, acyclischen oder cyclischen, aliphatischen Rest mit 1 bis 12 Kohlenstoffatomen enthält, wobei einzelne Kohlenstoffatome auch durch >P- substituiert sein können. Im Sinne von verzweigten cyclischen aliphatischen Resten sind damit eingeschlossen auch Reste wie beispielsweise $-CH_2-C_6H_{11}$. Als geeignete Reste sind beispielsweise genannt Methyl, Ethyl, 1-Propyl, 2-Propyl, 1-Butyl, 2-Butyl, 1-(2-Methyl)propyl, 2-(2-Methyl)propyl, 1-Pentyl, 1-Hexyl, 1-Heptyl, 1-Octyl, 1-Nonyl, 1-Decyl, 1-Undecyl, 1-Dodecyl, Cyclopentyl, Cyclohexyl, Cycloheptyl und Cyclooctyl, Methylcyclopentyl, Methylcyclohexyl, 1-(2-Methyl)-pentyl 1-(2-Ethyl)-hexyl, 1-(2-Propyl)heptyl und Norbonyl. Bevorzugt enthält der unverzweigte oder verzweigte, acyclische oder cyclische, aliphatische Rest mindestens 1 sowie bevorzugt maximal 10 Kohlenstoffatome. Im Falle eines ausschließlich cyclischen Restes im oben genannten Sinne beträgt die Zahl der Kohlenstoffatome 3 bis 12 und bevorzugt mindestens 4 sowie bevorzugt maximal 8 Kohlenstoffatome. Bevorzugte Reste sind 1-Butyl, 1-Octyl und Cyclohexyl.

Die Phosphin-Gruppe kann einen, zwei oder drei der oben genannten unverzweigten oder verzweigten, acyclischen oder cyclischen, aliphatischen Reste enthalten. Diese können gleich oder verschieden sein. Bevorzugt enthält die Phosphin-Gruppe drei der oben genannten unverzweigten oder verzweigten, acyclischen oder cyclischen, aliphatischen Reste, wobei besonders bevorzugt alle drei Reste gleich sind. Bevorzugte Phosphine sind $P(n-C_nH_{2n+1})_3$ mit n gleich 1 bis 10, besonders bevorzugt Tri-n-butylphosphin, Tri-n-octylphosphin, ganz besonders bevorzugt Tri-n-butylphosphin und Tri-n-octylphosphin, und insbesondere Tri-n-butylphosphin.

Wie bereits weiter oben erwähnt, können in den genannten unverzweigten oder verzweigten, acyclischen oder cyclischen, aliphatischen Resten einzelne Kohlenstoffatome auch durch >P- substituiert sein. Somit sind auch mehrzähnige, beispielsweise zwei- oder dreizähnige, Phosphin-Liganden mit umfasst. Diese enthalten bevorzugt die Gruppierung $>P-CH_2CH_2-P<$ beziehungsweise

$$>P-CH_2CH_2-\overset{|}{P}-CH_2CH_2-P< .$$

Enthält die Phosphin-Gruppe noch andere Reste als die oben genannten unverzweigten oder verzweigten, acyclischen

oder cyclischen, aliphatischen Reste, so entsprechen diese im Allgemeinen denen, die ansonsten üblicherweise bei Phosphin-Liganden für metallorganische Komplexkatalysatoren eingesetzt werden. Als Beispiele seien genannt Phenyl, Tolyl und Xylyl.

Der metallorganische Komplex kann eine oder mehrere, beispielsweise zwei, drei oder vier, der oben genannten Phosphin-Gruppen mit mindestens einem unverzweigten oder verzweigten, acyclischen oder cyclischen, aliphatischen Rest enthalten. Die restlichen Liganden des metallorganischen Komplexes können verschiedener Natur sein. Als exemplarische Beispiele seien genannt Hydrid, Fluorid, Chlorid, Bromid, Iodid, Formiat, Acetat, Propionat, Carboxylate, Acetylacetonat, Carbonyl, Dimethylsulfoxid, Hydroxid, Trialkylamin, Alkoxide.

Die homogenen Katalysatoren können sowohl direkt in ihrer aktiven Form als auch ausgehend von üblichen Standardkomplexen wie beispielsweise $[M(p\text{-Cymene})Cl_2]_2$, $[M(benzol)Cl_2]_n$, [M(COD)(allyl)], $[MCl_3 \times H_2O]$, $[M(acetylacetonat)_3]$, $[M(DMSO)_4Cl_2]$ mit M gleich Element aus der 8., 9. oder 10. Gruppe des Periodensystems unter Zugabe des beziehungsweise der entsprechenden Phosphin-Liganden erst unter Reaktionsbedingungen erzeugt werden.

Beim erfindungsgemäßen Verfahren bevorzugte homogene Katalysatoren sind $[Ru(P^nBu_3)_4(H)_2]$, $[Ru(P^nOctyl_3)_4(H)_2]$, $[Ru(P^nBu_3)_2$(1,2-bis(dicyclohexylphosphino)-ethan)$(H)_2]$, $[Ru(P^nOctyl_3)_2$(1,2-bis(dicyclohexylphosphino)ethan)$(H)_2]$. Mit diesen können in der Hydrierung von Kohlendioxid TOF-Werte (turn-over-frequency) von größer 1000 $h^{-1}$ erzielt werden.

Beim Einsatz von homogenen Katalysatoren beträgt die eingesetzte Menge der genannten Metallkomponente im metallorganischen Komplex im Allgemeinen 0,1 bis 5000 Gew.-ppm, bevorzugt 1 bis 800 Gew.-ppm und besonders bevorzugt 5 bis 500 Gew.-ppm, jeweils bezogen auf das gesamte flüssige Reaktionsgemisch im Hydrierreaktor.

Auch obwohl der homogene Katalysator in der Flüssigphase (B) angereichert vorliegt und somit bereits weitgehend über die Flüssigphase (B) wieder zum Hydrierreaktor rückgeführt wird, so enthält die mit dem Ameisensäure/Amin-Addukt (II) angereicherte Flüssigphase (A) in der Regel weiterhin noch wertvolle Restmengen an Katalysator. Diese könnten bei der thermischen Spaltung zu einer Rückreaktion unter Zersetzung in Kohlendioxid und Wasserstoff führen, was einem Verlust an Ameisensäure-Ausbeute gleichkommt. Zudem kann der durch die thermische Spaltung hindurchgeschleppte homogene Katalysator im Allgemeinen nicht ohne erneute Aktivierung oder Aufarbeitung in der Hydrierstufe eingesetzt werden. Daher ist beim erfindungsgemäßen Verfahren bevorzugt, dass man bei Merkmal (e) den Sumpf der Destillationseinheit in eine das freie tertiäre Amin (I) enthaltende Phase und eine das polare Lösungsmittel (III) enthaltende Phase trennt und beide Phasen getrennt zum Hydrierreaktor rückführt, wobei man die das freie tertiäre Amin (I) enthaltende Phase über eine Extraktionseinheit zum Hydrierreaktor rückführt und in der genannten Extraktionseinheit homogenen Katalysator aus der abgetrennten Flüssigphase (A) extrahiert, bevor das Ameisensäure/Amin-Addukt (II) der abgetrennten Flüssigphase (A) gemäß Merkmal (c) in einer Destillationseinheit thermisch in freie Ameisensäure und freies tertiäres Amin (I) gespalten wird.

Die Extraktion erfolgt im Allgemeinen bei einer Temperatur von 0 bis 150°C sowie einem Druck von 0,1 bis 8,0 MPa abs. Bevorzugt beträgt die Temperatur mindestens 20°C und besonders bevorzugt mindestens 30°C sowie bevorzugt höchstens 100°C und besonders bevorzugt höchstens 80°C. Der Druck beträgt bevorzugt mindestens 0,01 MPa abs und besonders bevorzugt mindestens 0,1 MPa abs sowie bevorzugt höchstens 10 MPa abs und besonders bevorzugt höchstens 1 MPa abs.

Die Extraktionseinheit umfasst dabei sowohl eine Vorrichtung zur Extraktion als auch eine Vorrichtung zur Trennung der beiden Flüssigphasen. Beide Teile können zusammen in einem Apparat integriert sein als auch auf mehrere Apparate aufgeteilt sein. Prinzipiell kann die genannte Extraktion in jeder geeigneten, dem Fachmann bekannten Vorrichtung durchgeführt werden. Bevorzugt sind der Einsatz einer Gegenstrom-Extraktionskolonne, einer Mixer-Settler-Kaskade oder einer Kombination von Mixer-Settlern mit Kolonnen.

Besonders vorteilhaft wird der zusätzliche Extraktionsschritt eingesetzt, wenn die mit dem Ameisensäure/Amin-Addukt (II) angereicherte Flüssigphase (A) noch mehr als 10 Gew.-ppm an Katalysatormetall enthält. Es ist jedoch auch bereits empfehlenswert, bei mehr als 1 Gew.-ppm an Katalysatormetall in der Flüssigphase (A) den zusätzlichen Extraktionsschritt einzusetzen.

Es kann vorteilhaft sein, zwischen der Extraktionseinheit und der Destillationseinheit, in der die thermische Spaltung erfolgt, eine Vorrichtung zur Adsorption von Spuren des homogenen Katalysators zu integrieren. Geeignete Adsorptionsmittel sind dem Fachmann bekannt. Als Beispiele geeigneter Adsorptionsmittel seien etwa Polyacrylsäure und deren Salze, sulfonierte Polystyrole und deren Salze, Aktivkohlen, Montmorillonite, Bentonite, Kieselgele sowie Zeolithe genannt.

Das beim erfindungsgemäßen Verfahren bei der Hydrierung von Kohlendioxid einzusetzende tertiäre Amin (I) weist bei einem Druck von 1013 hPa abs einen um mindestens 5°C höheren Siedepunkt als Ameisensäure auf. Das tertiäre Amin (I) ist dabei so auszuwählen beziehungsweise mit dem polaren Lösungsmittel (III) abzustimmen, dass das tertiäre Amin (I) in der Flüssigphase (B) angereichert vorliegt. Unter "angereichert" ist dabei ein Gewichtsanteil von > 50% des freien, das heißt nicht in Form des Ameisensäure/Amin-Addukts (II) gebundenen, tertiären Amins (I) in der Flüssigphase (B) bezogen auf die Gesamtmenge an freiem, tertiärem Amins (I) in beiden Flüssigphasen (A) und (B) zu verstehen. Bevorzugt liegt der Gewichtsanteil bei > 90%, besonders bevorzugt bei > 95% und ganz besonders bevorzugt

bei > 97%. Die Auswahl des tertiären Amins (I) erfolgt im Allgemeinen durch einen einfachen Versuch, in dem die Löslichkeit des gewünschten tertiären Amins (I) in beiden Flüssigphasen (A) und (B) unter den geplanten Verfahrensbedingungen experimentell bestimmt wird.

Bevorzugt weist das einzusetzende tertiäre Amin (I) einen um mindestens 10°C, besonders bevorzugt einem um mindestens 50°C und ganz besonders bevorzugt einem um mindestens 100°C höheren Siedepunkt als Ameisensäure auf. Eine Einschränkung hinsichtlich eines oberen Grenzwertes für den Siedepunkt ist nicht erforderlich, da für das erfindungsgemäße Verfahren ein möglichst niedriger Dampfdruck des tertiären Amin (I) grundsätzlich von Vorteil ist. Im Allgemeinen liegt der Siedepunkt des tertiären Amin (I) bei einem, gegebenenfalls nach bekannten Methoden vom Vakuum auf 1013 hPa abs hochgerechneten Drucks unterhalb von 500°C.

Das beim erfindungsgemäßen Verfahren bevorzugt einzusetzende tertiäre Amin (I) ist ein Amin der allgemeinen Formel (Ia)

$$NR^1R^2R^3 \qquad \text{(Ia)},$$

in der die Reste $R^1$ bis $R^3$ gleich oder verschieden sind und unabhängig voneinander einen unverzweigten oder verzweigten, acyclischen oder cyclischen, aliphatischen, araliphatischen oder aromatischen Rest mit jeweils 1 bis 16 Kohlenstoffatomen, bevorzugt 1 bis 12 Kohlenstoffatomen, darstellen, wobei einzelne Kohlenstoffatome unabhängig voneinander auch durch eine Heterogruppe ausgewählt aus der Gruppe -O- und >N- substituiert sein können sowie zwei oder alle drei Reste unter Bildung einer mindestens jeweils vier Atome umfassenden Kette auch miteinander verbunden sein können.

Als geeignete Amine sind beispielsweise genannt:

- Tri-n-propylamin ($Sdp_{1013\ hpa}$ = 156°C), Tri-n-butylamin, Tri-n-pentylamin, Tri-n-hexylamin, Tri-n-heptylamin, Tri-n-octylamin, Tri-n-nonylamin, Tri-n-decylamin, Tri-n-undecylamin, Tri-n-dodecylamin, Tri-n-tridecylamin, Tri-n-tetradecylamin, Tri-n-pentadecylamin, Tri-n-hexadecylamin, Tri-(2-ethylhexyl)amin.

- Dimethyl-decylamin, Dimethyl-dodecylamin, Dimethyl-tetradecylamin, Ethyl-di-(2-propyl)amin ($Sdp_{1013\ hPa}$ = 127°C), Dioctyl-methylamin, Dihexyl-methylamin.

- Tricyclopentylamin, Tricyclohexylamin, Tricycloheptylamin, Tricyclooctylamin und deren durch eine oder mehrere Methyl-, Ethyl-, 1-Propy-, 2-Propyl-, 1-Butyl-, 2-Butyl- oder 2-Methyl-2-propyl-Gruppen substituierte Derivate.

- Dimethyl-cyclohexylamin, Methyl-dicyclohexylamin, Diethyl-cyclohexylamin, Ethyldicyclohexylamin, Dimethyl-cyclopentylamin, Methyl-dicyclopentylamin.

- Triphenylamin, Methyl-diphenylamin, Ethyl-diphenylamin, Propyl-diphenylamin, Butyl-dipehnylamin, 2-Ethyl-hexyl-diphenylamin, Dimethyl-phenylamin, Diethyl-phenylamin, Dipropyl-phenylamin, Dibutyl-pehnylamin, Bis(2-Ethyl-hexyl)-phenylamin, Tribenzylamin, Methyl-dibenzylamin, Ethyl-dibenzylamin und deren durch eine oder mehrere Methyl-, Ethyl-, 1-Propy-, 2-Propyl-, 1-Butyl-, 2-Butyl- oder 2-Methyl-2-propyl-Gruppen substituierte Derivate.

- N-$C_1$-bis-$C_{12}$-Alkyl-piperidine, N,N-Di-$C_1$-bis-$C_{12}$-Alkyl-piperazine, N-$C_1$-bis-$C_{12}$-Alkyl-pyrrolidine, N-$C_1$-bis-$C_{12}$-rAlkyl-imidazole und deren durch eine oder mehrere Methyl- • Ethyl-, 1-Propy-, 2-Propyl-, 1-Butyl-, 2-Butyl- oder 2-Methyl-2-propyl-Gruppen substituierte Derivate.

- 1,8-Diazabicyclo[5.4.0]undec-7-en ("DBU"), 1,4-Diazabicyclo[2.2.2]octan ("DAB-CO"), N-Methyl-8-azabicyclo [3.2.1]octan ("Tropan"), N-Methyl-9-azabicyclo[3.3.1]nonan ("Granatan"), 1-Azabicyclo[2.2.2]octan ("Chinuclidin").

Beim erfindungsgemäßen Verfahren können natürlich auch Gemische verschiedener tertiärer Amine (I) eingesetzt werden.

Unter den zuvor beschriebenen tertiären Aminen der allgemeinen Formel (Ia) sind jene wiederum bevorzugt, bei denen die Reste $R^1$ bis $R^3$ gleich oder verschieden sind und unabhängig voneinander einen unverzweigten oder verzweigten, acyclischen oder cyclischen, aliphatischen, araliphatischen oder aromatischen Rest mit jeweils 1 bis 16 Kohlenstoffatomen, bevorzugt 1 bis 12 Kohlenstoffatomen, darstellen, wobei einzelne Kohlenstoffatome unabhängig voneinander auch durch eine Heterogruppe ausgewählt aus der Gruppe -O- und >N- substituiert sein können sowie zwei oder alle drei Reste unter Bildung einer mindestens jeweils vier Atome umfassenden, gesättigten Kette auch miteinander verbunden sein können.

Bevorzugt trägt mindestens einer der Reste am alpha-Kohlenstoffatom zwei Wasserstoffatome.

Besonders bevorzugt setzt man beim erfindungsgemäßen Verfahren als tertiäres Amin (I) ein Amin der allgemeinen Formel (Ia), in der die Reste $R^1$ bis $R^3$ unabhängig voneinander ausgewählt sind aus der Gruppe $C_1$- bis $C_{12}$-Alkyl, $C_5$- bis $C_8$-Cycloalkyl, Benzyl und Phenyl, ein.

Besonders bevorzugt setzt man beim erfindungsgemäßen Verfahren als tertiäres Amin (I) ein gesättigtes Amin der allgemeinen Formel (Ia) ein.

Ganz besonders bevorzugt setzt man beim erfindungsgemäßen Verfahren als tertiäres Amin (I) ein Amin der allgemeinen Formel (Ia) ein, in der die Reste $R^1$ bis $R^3$ unabhängig voneinander ausgewählt sind aus der Gruppe $C_5$- bis $C_8$-Alkyl, insbesondere Tri-n-pentylamin, Tri-n-hexylamin, Tri-n-heptylamin, Tri-n-octylamin, Dimethyl-cyclohexylamin, Methyl-dicyclohexylamin, Dioctyl-methylamin und Dimethyl-decylamin.

Bevorzugt liegt das tertiäre Amin (I) beim erfindungsgemäßen Verfahren in allen Verfahrensstufen flüssig vor. Dies ist jedoch kein zwingendes Erfordernis. Es würde auch ausreichen, wenn das tertiäre Amin (I) zumindest in geeigneten Lösungsmitteln gelöst wäre. Geeignete Lösungsmittel sind prinzipiell solche, welche hinsichtlich der Hydrierung von Kohlendioxid sowie der thermischen Spaltung des Addukts chemisch inert sind, in denen sich das tertiäre Amin (I) und der homogene Katalysator, sich gut lösen, umgekehrt polares Lösungsmittel (III) sowie das Ameisensäure/Amin-Addukt (II) schlecht lösen, und bei einem Druck von 1013 hPa abs einen um mindestens 5°C höheren Siedepunkt als Ameisensäure aufweisen. In Frage kommen daher prinzipiell chemisch inerte, unpolare Lösungsmittel wie etwa aliphatische, aromatische oder araliphatische Kohlenwasserstoffe, beispielsweise Octan und höhere Alkane, Toluol, Xylole.

Die Menge des beim erfindungsgemäßen Verfahren einzusetzenden tertiären Amins (I) beträgt im Allgemeinen 5 bis 95 Gew.-%, bevorzugt 20 bis 60 Gew.-% jeweils bezogen auf das gesamte flüssige Reaktionsgemisch im Hydrierreaktor.

Das beim erfindungsgemäßen Verfahren bei der Hydrierung von Kohlendioxid einzusetzende polare Lösungsmittel (III) besitzt einen elektrostatischen Faktor, auch EF abgekürzt, von $\geq 200 \cdot 10^{-30}$ Cm und weist bei einem Druck von 1013 hPa abs einen um mindestens 5°C höheren Siedepunkt als Ameisensäure auf. Das polare Lösungsmittel (III) ist dabei so auszuwählen beziehungsweise mit dem tertiären Amin (I) abzustimmen, dass das polare Lösungsmittel (III) in der Flüssigphase (A) angereichert vorliegt. Unter "angereichert" ist dabei ein Gewichtsanteil von > 50% des polaren Lösungsmittels (III) in der Flüssigphase (A) bezogen auf die Gesamtmenge an polarem Lösungsmittel (III) in beiden Flüssigphasen (A) und (B) zu verstehen. Bevorzugt liegt der Gewichtsanteil bei > 90%, besonders bevorzugt bei > 95% und ganz besonders bevorzugt bei > 97%. Die Auswahl des polaren Lösungsmittels (III) erfolgt im Allgemeinen durch einen einfachen Versuch, in dem die Lösichkeit des gewünschten polaren Lösungsmittels (III) in beiden Flüssigphasen (A) und (B) unter den geplanten Verfahrensbedingungen experimentell bestimmt wird.

Der elektrostatische Faktor EF ist definiert als das Produkt der relativen Dielektrizitätskonstanten $\varepsilon_r$ und dem Dipolmoment $\mu$ (siehe beispielsweise C.Reichardt, "Solvents and Solvent Effects in Organic Chemistry", 3. Auflage, Wiley-VCH Verlag GmbH & Co KGaA, Weinheim 2003, Kapitel 3.2, Seite 67 unten bis Seite 68 oben). Der genannte Mindestwert des elektrostatischen Faktors stellt sicher, dass das polare Lösungsmittel (III) eine gewisse Mindestpolarität aufweist, und sich das Ameisensäure/Amin-Addukt (II) bevorzugt in diesem löst.

Bevorzugt weist das einzusetzende polare Lösungsmittel (III) einen um mindestens 10°C, besonders bevorzugt einem um mindestens 50°C und ganz besonders bevorzugt einem um mindestens 85°C höheren Siedepunkt als Ameisensäure auf. Eine Einschränkung hinsichtlich eines oberen Grenzwertes für den Siedepunkt ist nicht erforderlich, da für das erfindungsgemäße Verfahren ein möglichst niedriger Dampfdruck des polaren Lösungsmittels (III) grundsätzlich von Vorteil ist. Im Allgemeinen liegt der Siedepunkt des polaren Lösungsmittels (III) bei einem, gegebenenfalls nach bekannten Methoden vom Vakuum auf 1013 hPa abs hochgerechneten Drucks unterhalb von 500°C.

Als Stoffklassen, die als polare Lösungsmittel (III) geeignet sind, kommen bevorzugt Diole sowie deren Ameisensäureester, Polyole sowie deren Ameisensäureester, Sulfone, Sulfoxide, offenkettige oder cyclische Amide sowie Gemische der genannten Stoffklassen in Frage.

Als geeignete Diole und Polyole sind beispielsweise Ethylenglykol, Diethylenglykol, Triethylenglykol, Polyethylenglykol, 1,3-Propandiol, 2-Methyl-1,3-propandiol, 1,4-Butandiol, Dipropylenglykol, 1,5-Pentandiol, 1,6-Hexandiol und Glycerin genannt. Diole und Polyole können aufgrund ihrer OH-Gruppen in Gegenwart von Ameisensäure verestert werden. Dies geschieht beim erfindungsgemäßen Verfahren vor allem bei der thermischen Spaltung des Ameisensäure/Amin-Addukts (II) in der genannten Destillationseinheit. Da die entstehenden Ameisensäureester ein sehr ähnliches Phasenverhalten zeigen, sind diese als polares Lösungsmittel ebenso gut geeignet. Auch das bei der Veresterung entstehende Wasser, welches durchaus bis zu 5 Gew.-% bezogen auf das gesamte Hydriergemisch betragen kann, ist nicht schädlich - weder bei der Hydrierung noch bei der thermischen Spaltung. Eine Aufpegelung des Wassers im kontinuierlichen Betrieb des erfindungsgemäßen Verfahrens erfolgt nicht, da Wasser in diesen geringen Mengen in der Destillationseinheit der thermischen Spaltung im Ameisensäure-Schwersiederazeotrop über einen Seitenabzug abgetrennt werden kann. Es kann gegebenenfalls sogar vorteilhaft sein, beim Einsatz von Diolen oder Polyolen noch zusätzlich Wasser zuzusetzen, um das Gleichgewicht zwischen den OH- und den Ameisensäureester-Gruppen in Richtung der OH-Gruppen zu verschieben. Im Falle eines Zusatzes von Wasser liegt die zugesetzte Wassermenge im Allgemeinen bei 0,1 bis 20 Gew.-% bezogen auf das gesamte flüssige Reaktionsgemisch im Hydrierreaktor.

Als geeignete Sulfoxide sind beispielsweise Dialkylsulfoxide, vorzugsweise $C_1$- bis $C_6$-Dialkylsulfoxide, insbe-

sondere Dimethylsulfoxid genannt.

Als geeignete offenkettige oder cyclische Amide sind beispielsweise Formamid, N-Methylformamid N,N-Dimethylformamid, N-Methylpyrrolidon, Acetamid und N-Methylcaprolactam genannt.

Bevorzugt setzt man beim erfindungsgemäßen Verfahren als polares Lösungsmittel (III) einen aliphatischen, gesättigten Kohlenwasserstoff mit 2 bis 5 OH-Gruppen sowie deren Ameisensäureester ein. Als besonders bevorzugte Diole und Polyole sind genannt Ethylenglykol, 1,4-Butandiol, 2-Methyl-1,3-propandiol, und deren Ameisensäureester.

Das Molverhältnis des beim erfindungsgemäßen Verfahren einzusetzenden polaren Lösungsmittels (III) zum eingesetzten tertiären Amin (I) beträgt im Allgemeinen 0,5 bis 30 und bevorzugt 2 bis 20.

Es ist allgemein bekannt, dass OH-Gruppen enthaltende Verbindungen die Hydrierung von Kohlendioxid beschleunigen. So lehren etwa EP 0 095 321 A, EP 0 151 510 A, EP 0 181 078 A, EP 0 357 234 A und DE 44 31 233 A den Zusatz von Wasser. Auch beim vorliegenden erfindungsgemäßen Verfahren ist der Einsatz von OH-Gruppen enthaltende Verbindungen als Promotor für die Hydrierung von Kohlendioxid grundsätzlich bevorzugt. Da allerdings die oben genannten Diole und Polyole bereits ebenfalls einen derartigen positiven Effekt ausüben, ist bei diesen der Zusatz weiterer OH-Gruppen enthaltender Verbindungen im Allgemeinen nicht weiter förderlich. Anders hingegen beim Einsatz anderer polarer Lösungsmittel (III), welche keine OH-Gruppen enthalten, wie beispielsweise Ameisensäureester, Sulfone, Sulfoxide oder offenkettige oder cyclische Amide. In diesen Fällen ist es vorteilhaft, Wasser und/oder Alkohole, beispielsweise aliphatische, gesättigte Monoalkohole zuzusetzen. Im Allgemeinen reicht ein Zusatz von 1 bis 500 mmol pro mg des Gruppe 8 bis 10 Metallkatalysators.

Das bei der Hydrierung von Kohlendioxid einzusetzende Kohlendioxid kann fest, flüssig oder gasförmig eingesetzt werden. Es ist auch möglich, großtechnisch verfügbare, Kohlendioxid enthaltende Gasgemische zu verwenden, sofern diese weitgehend frei von Kohlenmonoxid sind. Der bei der Hydrierung von Kohlendioxid einzusetzende Wasserstoff ist im Allgemeinen gasförmig. Kohlendioxid und Wasserstoff können auch inerte Gase, wie etwa Stickstoff oder Edelgase, enthalten. Vorteilhafterweise liegt jedoch deren Gehalt unterhalb von 10 mol-% bezogen auf die Gesamtmenge an Kohlendioxid und Wasserstoff im Hydrierreaktor. Größere Mengen sind zwar gegebenenfalls ebenfalls noch tolerierbar, bedingen aber im Allgemeinen die Anwendung eines höheren Druckes im Reaktor, wobei dadurch weitere Kompressionsenergie erforderlich ist.

Die Hydrierung von Kohlendioxid erfolgt in der Flüssigphase bei einer Temperatur von 20 bis 200°C und einem Gesamtdruck von 0,2 bis 30 MPa abs. Bevorzugt beträgt die Temperatur mindestens 30°C und besonders bevorzugt mindestens 40°C sowie bevorzugt höchstens 150°C, besonders bevorzugt höchstens 120°C und ganz besonders bevorzugt höchstens 80°C. Der Gesamtdruck beträgt bevorzugt mindestens 1 MPa abs und besonders bevorzugt mindestens 5 MPa abs sowie bevorzugt höchstens 20 MPa abs, besonders bevorzugt höchstens 15 MPa abs und insbesondere höchstens 10 MPa abs.

Der Partialdruck des Kohlendioxids beträgt dabei im Allgemeinen mindestens 0,5 MPa und bevorzugt mindestens 2 MPa sowie im Allgemeinen höchstens 6 MPa und bevorzugt höchstens 5 MPa. Der Partialdruck des Wasserstoffs beträgt im Allgemeinen mindestens 0,5 MPa und bevorzugt mindestens 1 MPa sowie im Allgemeinen höchstens 250 MPa und bevorzugt höchstens 10 MPa.

Das Molverhältnis von Wasserstoff zu Kohlendioxid im Zulauf des Hydrierreaktors beträgt bevorzugt 0,1 bis 10 und besonders bevorzugt 1 bis 3.

Das Molverhältnis von Kohlendioxid zu tertiärem Amin (I) im Zulauf des Hydrierreaktors beträgt im Allgemeinen 0,1 bis 10 und bevorzugt 0,5 bis 3.

Als Hydriereaktoren können prinzipiell alle Reaktoren eingesetzt werden, welche für gas/flüssig-Reaktionen unter der gegebenen Temperatur und des gegebenen Drucks grundsätzlich geeignet sind. Geeignete Standardreaktoren für flüssig-flüssig Reaktionssyteme sind beispielsweise in K.D. Henkel, "Reactor Types and Their Industrial Applications", in Ullmann's Encylcopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien Rührkesselreaktoren, Rohrreaktoren oder Blasensäulenreaktoren genannt.

Die Hydrierung von Kohlendioxid kann beim erfindungsmäßen Verfahren diskontinuierlich oder kontinuierlich durchgeführt werden. Bei der diskontinuierlichen Fahrweise wird der Reaktor mit den gewünschten flüssigen und gegebenenfalls festen Einsatz- und Hilfsstoffen bestückt und anschließend Kohlendioxid und Wasserstoff auf den gewünschten Druck bei der gewünschten Temperatur aufgepresst. Nach Ende der Reaktion wird der Reaktor im Regelfall entspannt und die beiden gebildeten Flüssigphasen (A) und (B) voneinander getrennt. Bei der kontinuierlichen Fahrweise werden die Einsatz- und Hilfsstoffe, einschließlich des Kohlendioxids und Wasserstoffs kontinuierlich zugegeben. Entsprechend wird die Fiüssigphase kontinuierlich aus dem Reaktor abgeführt, so dass der Flüssigkeitsstand im Reaktor im Mittel gleich bleibt. Bevorzugt ist die kontinuierliche Hydrierung von Kohlendioxid.

Die mittlere Verweilzeit im Reaktor beträgt im Allgemeinen 10 Minuten bis 5 Stunden.

Das bei der Hydrierung von Kohlendioxid in Gegenwart des einzusetzenden homogenen Katalysators und des tertiären Amins (I) gebildete Ameisensäure/Amin-Addukt (II) weist im Allgemeinen die allgemeine Formel (IIa)

$NR^1R^2R^3 \cdot x\ HCOOH$ (IIa),

auf, in der die Reste $R^1$ bis $R^3$ den beim tertiären Amin (Ia) beschriebenen Resten bedeuten und x gleich 0.5 bis 5, bevorzugt 0.7 bis 1.5 beträgt. Der Faktor x lässt sich beispielsweise durch Titration mit einer alkoholischen KOH-Lösung gegen Phenolphthalein bestimmen. Die genaue Zusammensetzung des Ameisensäure/Amin-Addukt (II) ist von vielen Parametern abhängig, wie beispielsweise den vorliegenden Konzentrationen von Ameisensäure und tertiärem Amin (I), Druck, Temperatur oder der Gegenwart und Natur weiterer Komponenten, insbesondere eines polaren Lösungsmittels (III). Daher kann sich die Zusammensetzung des Ameisensäure/Amin-Addukt (II) auch über die einzelnen Verfahrensstufen hinweg ändern, wobei im Rahmen der vorliegenden Patentanmeldung jeweils von dem Ameisensäure/Amin-Addukt (II) die Rede ist. Die Zusammensetzung des Ameisensäure/Amin-Adduktes (II) lässt sich in jeder Verfahrensstufe leicht durch Bestimmung des Ameisensäuregehaltes per Säure-Base-Titration und Bestimmung des Amingehaltes per Gaschromatographie ermitteln.

Bei der Hydrierung von Kohlendioxid nach dem erfindungsgemäßen Verfahren werden zwei Flüssigphasen gebildet. Flüssigphase (A) ist mit dem Ameisensäure/Amin-Addukt (II) sowie dem polaren Lösungsmittel (III) angereichert. Hinsichtlich des Ameisensäure/Amin-Addukts (II) ist unter "angereichert" ein Verteilungskoeffizient des Ameisensäure/Amin-Addukts (II)

P = [Konzentration Ameisensäure/Amin-Addukt (II) in Flüssigphase (A)] / [Konzentration Ameisensäure/Amin-Addukt (II) in Flüssigphase (B)]

von > 1 zu verstehen. Bevorzugt liegt der Verteilungskoeffizient bei > 2 und besonders bevorzugt bei ≥ 5. Die Flüssigphase (B) ist mit dem tertiären Amin (I) und dem homogenen Katalysator angereichert.

Die beiden gebildeten Flüssigphasen (A) und (B) werden beim erfindungsgemäßen Verfahren voneinander getrennt und die Flüssigphase (B) zum Hydrierreaktor rückgeführt. Gegebenenfalls ist auch eine Rückführung einer über beiden Flüssigphasen vorhandenen weiteren Flüssigphase enthaltend nicht umgesetztes Kohlendioxid sowie einer Gasphase enthaltend nicht umgesetztes Kohlendioxid und/oder nicht umgesetzten Wasserstoff zum Hydrierreaktor vorteilhaft. Gegebenenfalls ist es beispielsweise zur Ausschleusung von unerwünschten Nebenprodukten oder Verunreinigungen wünschenswert, einen Teil der Flüssigphase (B) und/oder einen Teil der Kohlendioxid oder Kohlendioxid und Wasserstoff enthaltenden flüssigen beziehungsweise gasförmigen Phasen aus dem Verfahren auszuschleusen.

Die Trennung der beiden Flüssigphasen (A) und (B) erfolgt im Allgemeinen durch gravimetrische Phasentrennung. Geeignet hierzu sind beispielsweise Standardapparaturen und Standardmethoden, welche sich zum Beispiel in E. Müller et al., "Liquid-Liquid Extraction", in Ullmann's Encylcopedia of Industrial Chemistry, 2005, Wiley-VCH Verlag GmbH & Co. KGaA, DOI:10.1002/14356007.b03_06, Kapitel 3 "Apparatus" finden lassen. Im Allgemeinen ist die mit dem Ameisensäure/Amin-Addukt (II) sowie dem polaren Lösungsmittel (III) angereicherte Flüssigphase (A) schwerer und bildet die untere Phase.

Die Phasentrennung kann beispielsweise nach Entspannung, beispielsweise auf etwa oder nahe Atmosphärendruck, und Abkühlung des flüssigen Reaktionsgemischs, beispielsweise auf etwa oder nahe Umgebungstemperatur, erfolgen. Allerdings ist dabei zu befürchten, dass zumindest ein Teil des bei dem höherem Reaktionsdruck in den Flüssigphasen gelöste Gas, insbesondere Kohlendioxid, bei der Entspannung entgast und als Gasstrom separat zu verdichten und zum Hydrierreaktor rückzuführen ist. Ebenso ist auch die Flüssigphase (B) vor der Rückführung zum Hydrierreaktor separat zu verdichten. Beide Verdichterstufen für die rückzuführende Gas- und Flüssigphase bedürfen jeweils eines geeigneten, entsprechend der zu überwindenden Druckdifferenz ausgelegten Verdichters und verbrauchen im Betrieb zudem zusätzliche Energie.

Im Rahmen der vorliegenden Erfindung wurde überraschend gefunden, dass sich bei dem vorliegenden System, das heißt, einer mit dem Ameisensäure/Amin-Addukt (II) sowie dem polaren Lösungsmittel (III) angereicherten Flüssigphase (A) und einer mit dem tertiären Amin (I) und den homogenen Katalysator angereicherten Flüssigphase (B), beide Flüssigphasen auch bei einem deutlich erhöhten Druck sehr gut voneinander trennen. Daher ist beim erfindungsgemäßen Verfahren die Trennung der einen, mit dem Ameisensäure/Amin-Addukt (II) und dem polaren Lösungsmittel (III) angereicherten Flüssigphase (A) von der anderen, mit dem tertiären Amin (I) angereicherten Flüssigphase (B), sowie die Rückführung der Flüssigphase (B) zum Hydrierreaktor bei einem Druck von 1 bis 30 MPa abs bevorzugt. Gemäß dem Gesamtdruck im Hydrierreaktor, liegt der Druck bei bevorzugt höchstens 15 MPa abs und besonders bevorzugt höchstens 10 MPa abs. So ist es sogar möglich, ohne vorhergehende Entspannung beide Flüssigphasen voneinander zu trennen und die Flüssigphase (B) ohne nennenswerte Druckerhöhung zum Hydrierreaktor zurückzuführen. In diesem Falle, sowie auch im Falle einer nur geringfügigen Entspannung ist es möglich, auf eine Rückführung einer etwaigen Gasphase gänzlich zu verzichten. Ob dieser Verzicht für das jeweils konkrete System möglich ist, ist im Zweifel vorab durch einfache experimentelle Beispiele zu ermitteln.

Besonders bevorzugt erfolgt die Phasentrennung bei einem Druck von mindestens 50%, ganz besonders

bevorzugt von mindestens 90% und insbesondere von mindestens 95% des Reaktionsdrucks. Der Druck bei der Phasentrennung liegt besonders bevorzugt bei höchstens 105% und ganz besonders bevorzugt bei höchstens 100% des Reaktionsdrucks.

Überraschenderweise wurde auch gefunden, dass sich bei dem vorliegenden System beide Flüssigphasen auch bei einer erhöhten Temperatur, die der Reaktionstemperatur entspricht, sehr gut voneinander trennen. Insofern ist zur Phasentrennung auch keine Abkühlung und anschließende Aufheizung der rückzuführenden Flüssigphase (B) erforderlich, was ebenfalls Energie einspart.

Die Erkenntnisse zur Phasentrennung unter erhöhtem Druck und erhöhter Temperatur werden noch dadurch übertroffen, dass gerade die Flüssigphase (B) gemäß des erfindungsgemäßen Systems unter Druck eine besonders hohe Aufnahmekapazität für Kohlendioxid aufweist. Dies heißt, dass eventuell überschüssiges, in der Hydrierreaktion nicht umgesetztes Kohlendioxid stark bevorzugt in der Flüssigphase (B) vorliegt und somit problemlos als Flüssigkeit zum Reaktor rückgeführt werden kann.

Das Ameisensäure/Amin-Addukt (II) der abgetrennten Flüssigphase (A) wird dann in einer Destillationseinheit thermisch in freie Ameisensäure und freies tertiäres Amin (I) gespalteten, wobei die gebildete freie Ameisensäure destillativ entfernt und das im Sumpf der Destillationseinheit enthaltene freie tertiäre Amin (I) sowie das polare Lösungsmittel (III) zum Hydrierreaktor rückführt wird. Die Entnahme der freigesetzten Ameisensäure kann dabei beispielsweise (i) über Kopf, (ii) über Kopf und als Seitenabzug oder (iii) nur als Seitenabzug erfolgen. Wird Ameisensäure über Kopf entnommen, so ist dabei eine Ameisensäure-Reinheit von bis zu 99,9 Gew.-% möglich. Bei einer Entnahme als Seitenabzug wird wässrige Ameisensäure erhalten, wobei hierbei das Gemisch mit etwa 85 Gew.-% Ameisensäure in der Praxis von besonderer Bedeutung ist. Je nach dem Wassergehalt des Zulaufs zur Destillationseinheit wird die Ameisensäure verstärkt als Kopfprodukt oder als Seitenprodukt entnommen. Bei Bedarf ist es sogar möglich, Ameisensäure nur als Seitenprodukt zu entnehmen, wobei dann gegebenenfalls die erforderliche Wassermenge sogar noch explizit zugegeben werden kann. Die thermische Spaltung des Ameisensäure/Amin-Addukts (II) erfolgt im Allgemeinen nach den im Stand der Technik bekannten Verfahrensparametern hinsichtlich Druck, Temperatur sowie apparativer Ausgestaltung. So sei beispielsweise auf die Beschreibungen in EP 0 181 078 A oder WO 2006/021,411 verwiesen. Die einzusetzende Destillationseinheit umfasst in der Regel eine Destillationskolonne, welche im Allgemeinen Füllkörper, Packungen und/oder Glockenböden enthält.

Das aus der Destillationseinheit entnommene Sumpfprodukt kann noch geringe Restmengen an Ameisensäure enthalten, wobei das Molverhältnis von Ameisensäure zu tertiärem Amin (I) bevorzugt jedoch $\leq$ 0,1 und besonders bevorzugt $\leq$ 0,05 beträgt.

Im Allgemeinen beträgt die Sumpftemperatur in der Destillationskolonne mindestens 130°C, bevorzugt mindestens 150°C und besonders bevorzugt mindestens 170°C, sowie im Allgemeinen höchstens 210°C, bevorzugt höchstens 190°C und besonders bevorzugt höchstens 185°C. Der Druck beträgt im Allgemeinen mindestens 1 hPa abs, bevorzugt mindestens 50 hPa abs und besonders bevorzugt mindestens 100 hPa abs, sowie im Allgemeinen höchstens 500 hPa abs, bevorzugt höchstens 300 hPa abs und besonders bevorzugt höchstens 250 hPa abs.

Gegebenenfalls wird als Seitenprodukt noch ein wasserhaltiger Strom an Ameisensäure entnommen. Bei einem Zusatz von Wasser, beispielsweise zur Förderung der Hydrierung, ist dies sogar besonders vorteilhaft.

DE 34 28 319 A beschreibt bereits die thermische Spaltung eines Addukts zwischen Ameisensäure und einem tertiären Amin mit $C_6$- bis $C_{14}$-Alkylresten in einer Spaltkolonne. Ebenso beschreibt auch WO 2006/021,411 die thermische Spaltung eines Addukts zwischen Ameisensäure und einem tertiären Amin mit einem Siedepunkt bei Normaldruck von 105 bis 175°C in einer Spaltkolonne. EP 0 563 831 A offenbart desgleichen die thermische Spaltung eines Addukts zwischen Ameisensäure und einem tertiären Amin mit einem höheren Siedepunkt als Ameisensäure, wobei zugesetztes Formamid eine besonders farbzahlstabile Ameisensäure ergeben soll.

Abbildung 1 zeigt ein schematisches Blockdiagramm einer möglichen Ausführungsform des erfindungsgemäßen Verfahrens. Darin haben die einzelnen Buchstaben folgende Bedeutung:

A = Hydrierreaktor
B = Phasenabscheider
C = Destillationseinheit

Kohlendioxid und Wasserstoff werden in den Hydrierreaktor "A" geführt. Darin werden diese in Gegenwart eines homogenen Katalysators enthaltend ein Element aus der 8., 9. oder 10. Gruppe des Periodensystems, eines tertiären Amins (I) und eines polaren Lösungsmittels (III) zu einem Ameisensäure/Amin-Addukt (II) umgesetzt. Dabei bilden sich die beiden Flüssigphasen (A) und (B) aus. Flüssigphase (A) ist mit dem Ameisensäure/Amin-Addukt (II) sowie dem polaren Lösungsmittel (III) angereichert, Flüssigphase (B) mit dem tertiären Amin (I) und den homogenen Katalysator angereichert. Beide Flüssigphasen werden einem Phasenabscheider "B" zugeführt und voneinander getrennt. Flüssigphase (B), welche im Allgemeinen die obere Phase ist, wird zum Hydrierreaktor "A" rückgeführt. Flüssigphase (A) wird einer Destillationseinheit "C" zugeführt und das im Hydrierreaktor "A" gebildete Ameisensäure/Amin-Addukt (II) darin in freie Ameisensäure und tertiäres Amin (I) thermisch gespalten. Die freie Ameisensäure

wird beispielsweise als Kopfprodukt entfernt. Der Sumpf der Destillationseinheit "C" wird zum Hydrierreaktor "A" rückgeführt.

Selbstverständlich ist es möglich, das erfindungsgemäße Verfahren bei Bedarf durch weitere Verfahrensschritte beziehungsweise Zu- oder Abläufe von Stoffströmen zu ergänzen. Als nicht limitierendes Beispiel seien etwa genannt die Zufuhr von Hilfsstoffen wie zum Beispiel tertiäres Amin (I), polares Lösungsmittel (III), homogener Katalysator oder Wasser zur Aufrechterhaltung ihrer Konzentrationen im Verfahren.

Abbildung 2 zeigt ein schematisches Blockdiagramm einer bevorzugten Ausführungsform des erfindungsgemäßen Verfahrens. Darin haben die einzelnen Buchstaben folgende Bedeutung:

A = Hydrierreaktor
B = Phasenabscheider
C = Destillationskolonne
D = Phasenabscheider
E = Extraktionsvorrichtung
F = Phasenabscheider

Hinsichtlich der Verfahrensdurchführung in den Apparaten "A" und "B" gilt das für Abbildung 1 Beschriebene. Auch bei dem bevorzugten Verfahren nach Abbildung 2 wird die Flüssigphase (B), welche im Allgemeinen die obere Phase ist, zum Hydrierreaktor "A" rückgeführt. Die Flüssigphase (A) wird hier jedoch vor der Zuführung zur Destillationskolonne "C" erst noch einer Extraktion mit rückzuführendem tertiärem Amin (I) zur weiteren Abtrennung von homogenem Katalysator unterworfen. Diese erfolgt in der Extraktionsvorrichtung "E" mit nachfolgender Phasentrennung im Phasenabscheider "F". In diesem trennen sich die beiden Flüssigphasen (C) und (D). Die Flüssigphase (C), welche die mit dem homogenen Katalysator angereicherte Aminphase ist und im Allgemeinen die obere Phase darstellt, wird dem Hydrierreaktor "A" zugeführt. Die Flüssigphase (D), welche mit dem Ameisensäure/Amin-Addukt (II) sowie dem polaren Lösungsmittel (III) angereichert ist, wird der Destillationskolonne "C" zugeführt und darin das Ameisensäure/Amin-Addukt (II) in freie Ameisensäure und tertiäres Amin (I) thermisch gespalten. Die freie Ameisensäure wird beispielsweise als Kopfprodukt entfernt. Der Sumpf der Destillationskolonne "C" wird im Phasenabscheider "D" in zwei Flüssigphasen (E) und (F) getrennt. Die Flüssigphase (E), welche hauptsächlich das tertiäre Amin (I) enthält und im Allgemeinen die obere Phase darstellt, wird der Extraktionsvorrichtung "E" zugeführt. Die Flüssigphase (F), welche hauptsächlich das polare Lösungsmittel (III) enthält, wird zum Hydrierreaktor "A" rückgeführt.

Das erfindungsgemäße Verfahren ermöglicht die Gewinnung von konzentrierter Ameisensäure in hoher Ausbeute und hoher Reinheit durch Hydrierung von Kohlendioxid. Es zeichnet sich insbesondere durch eine besonders einfache und geschickte Verfahrensführung aus, welche gegenüber dem Stand der Technik ein einfacheres Verfahrenskonzept, einfachere Verfahrensstufen, eine geringere Anzahl an Verfahrensstufen sowie einfachere Apparate aufweist. So wird beispielsweise durch geeignete Wahl des tertiären Amins (I) und des polaren Lösungsmittels der homogene Katalysator durch Phasentrennung vom Ameisensäure/Amin-Addukt (II) getrennt und ohne weitere Aufarbeitungsschritte zur Hydrierreaktor rückgeführt. Aufgrund der zügigen Abtrennung des Katalysators vom gebildeten Ameisensäure/Amin-Addukt (II) wird eine Rückreaktion unter Zersetzung in Kohlendioxid und Wasserstoff unterdrückt. Zudem werden durch die Rückhaltung beziehungsweise Abtrennung des Katalysators aufgrund der Ausbildung zweier Flüssigphasen Verluste an Katalysator und damit Verluste an Edelmetall minimiert. Ferner ist beim erfindungsgemäßen Verfahren kein aufwändiger, separater Basenaustausch erforderlich, so dass das in der Hydrierreaktor gebildete Ameisensäure/Amin-Addukt (II) direkt zur thermischen Spaltung eingesetzt werden kann. Das dabei freigewordene tertiäre Amin (I) wird wieder zum Hydrierreaktor rückgeführt. Diese Phasentrennung kann sogar unter Druck erfolgen. Als Folge des einfacheren Verfahrenskonzepts ist die zur Durchführung des erfindungsgemäßen Verfahrens erforderliche Produktionsanlage gegenüber dem Stand der Technik kompakter im Sinne eines geringeren Platzbedarfs und des Einsatz von wenigeren Apparaten. Sie weist einen geringeren Investitionsaufwand und einen geringeren Energiebedarf auf.

Durch eine zusätzliche Extraktion des Ameisensäure/Amin-Addukt (II) haltigen Stroms vor dessen Zufuhr zur thermischen Spaltung kann eine weitere Abreicherung des Katalysators mit dem rückzuführenden tertiären Amin (I) erfolgen. Aufgrund des dadurch noch niedrigeren Gehalts an homogenem Katalysator in dem zu spaltenden Ameisensäure/Amin-Addukt (II) wird eine mögliche Rückreaktion bei der Spaltung unter Zersetzung in Kohlendioxid und Wasserstoff noch besser unterdrückt. Zudem wird durch die dadurch erhöhte Rückführrate des homogenen Katalysators auch dessen Nachschub in Form von frischem Katalysator weiter verringert.

Beispiele

Falls nicht anders vermerkt, handelt es sich bei den genannten Trialkylaminen jeweils um die entsprechenden Tri-n-alkylamine.

Beispiele A-1 bis A-27 (Hydrierung und Phasentrennung)

Sofern nicht anders vermerkt, wurde ein 250 mL Autoklav aus Hastelloy C, ausgestattet mit einem Magnetrührstab, unter Inertbedingungen mit tertiärem Amin, polarem Lösungsmittel und homogenem Katalysator befüllt, wobei ein zweiphasiges Gemisch erhalten wurde (obere Phase: Amin und Katalysator; untere Phase: Lösungsmittel). Anschließend wurde der Autoklav verschlossen und bei Raumtemperatur $CO_2$ aufgepresst. Nachfolgend wurde $H_2$ aufgepresst und der Reaktor unter Rühren (700 U/min) erwärmt. Nach der gewünschten Reaktionszeit wurde der Autoklav abgekühlt und das Reaktionsgemisch entspannt. Es wurde - sofern nicht anders vermerkt - ein zweiphasiges Produktgemisch erhalten, wobei die obere Phase mit dem noch freien tertiären Amin und dem homogenen Katalysator, und die untere Phase mit dem polaren Lösungsmittel und dem gebildeten Ameisensäure/Amin-Addukt angereichert war. Bei einigen Beispielen wurde der Verteilungskoeffizient K des Rutheniums

$$K = [\text{Ru-Konzentration in oberer Phase}] / [\text{Ru-Konzentration in unterer Phase}]$$

mittels Atomadsorptionsspektrometrie (AAS) bestimmt. Der Gesamtgehalt an Ameisensäure im Ameisensäure/Amin-Addukt wurde durch Titration mit 0,1 N KOH in 2-Propanol gegen Phenolphthalein unter Abzug des Blindwertes bestimmt. Daraus wurden die TON, die TOF und die Reaktionsrate berechnet. Die Parameter und Ergebnisse der einzelnen Versuche sind in Tabelle 1.1 bis 1.7 wiedergegeben.

Der Blindwert basiert darauf, dass sich geringe Mengen Kohlendioxid auch in der Ameisensäure/Amin-Addukt haltigen Phase lösen und durch KOH gegen Phenolphthalein titriert werden können. Die Bestimmung des Blindwertes erfolgte für jedes Beispiel separat durch einen völlig analogen Blindversuch, bei dem lediglich der homogene Katalysator weggelassen und am Ende die emulgierte Gesamtprobe wie oben beschrieben titriert wurde.

Die Beispiele A-1 bis A-27 zeigen, dass beim erfindungsgemäßen Verfahren selbst unter Variation des tertiären Amins, des polaren Lösungsmittels, des Katalysators hinsichtlich der Liganden und der Metallkomponente, der Menge des Katalysators, sowie auch unter zusätzlicher Zugabe von Wasser hohe bis sehr hohe Reaktionsraten von sogar bis über 1 mol $kg^{-1}$ $h^{-1}$ erzielt werden können. Alle untersuchten Systeme bildeten zwei Phasen, wobei die obere Phase jeweils mit dem noch freien tertiären Amin und dem homogenen Katalysator, und die untere Phase jeweils mit dem polaren Lösungsmittel und dem gebildeten Ameisensäure/Amin-Addukt angereichert war.

Beispiele B-1 bis B-12 (Phasenverhalten und $CO_2$-Löslichkeit unter Druck)

In diesen Beispielen wurde das Phasenverhalten von Gemischen enthaltend tertiäres Amin, polares Lösungsmittel und Ameisensäure/Amin-Addukt in Bezug auf die spezifische Löslichkeit von $CO_2$ unter Druck untersucht. Hierzu wurden zu einem zweiphasigen Gemisch jeweils bestehend aus 10,0 g polarem Lösungsmittel und 10,0 g tertiärem Amin unter kräftigem Rühren 0,5 g Ameisensäure gegeben. Die Ameisensäure reagierte jeweils mit dem tertiären Amin unter Bildung des entsprechenden Ameisensäure/Amin-Addukts, welches sich jeweils in der Phase des polaren Lösungsmittel löste. Von der jeweils erhaltenen Emulsion wurden 4 mL in eine Hochdruck-Sichtzelle gegeben und abgewartet, bis sich beide Phasen voneinander trennten. Anschließend wurden die Volumenstände der beiden Flüssigkeitsphasen markiert und bei 20°C auf 6,5 MPa abs $CO_2$ aufgepresst. Es wurde jeweils solange $CO_2$ nachgeliefert, bis sich ein konstanter Druck von 6,5 MPa abs einstellte. Die Volumenstände der beiden Flüssigkeitsphasen wurden nach 15 Minuten nun erneut markiert und mit den ursprünglichen, vor der Einpressung des $CO_2$, verglichen. Ein Anstieg des Phasenvolumens ist auf eine Einlösung von $CO_2$ zurückzuführen. Die Parameter und Ergebnisse der einzelnen Versuche sind in Tabelle 2.1 bis 2.3 wiedergegeben.

Zum Einen zeigen die Beispiele, dass selbst bei hohem Druck die Zweiphasigkeit vieler Systeme beibehalten wird und somit auch bei 6,5 MPa abs noch problemlos eine Phasentrennung möglich ist. Zum Anderen zeigen die Beispiele durch die Volumenzunahme der oberen, mit dem tertiären Amin angereicherten Flüssigphase (B), dass viele der getesteten tertiären Amine eine hohe $CO_2$-Aufnahmekapazität besitzen.

Bei der bevorzugten Ausführungsform der Phasentrennung und Rückführung der Flüssigphase (B) zum Hydrierreaktor unter Druck ist dies von besonderem Vorteil, da das $CO_2$ gelöst zurückgeführt werden kann. Zudem ist eine hohe $CO_2$-Löslichkeit in der Flüssigphase (B) auch für die Hydrierung von besonderem Vorteil, da der homogene Katalysator ja gerade der Flüssigphase (B) vorliegt und somit in dessen Umgebung eine hohe Konzentration an gelöstem $CO_2$ als Edukt vorliegt.

Beispiele C-1 bis C-34 (Verteilung der homogenen Katalysatoren)

In diesen Beispielen wurde die Verteilung verschiedener homogener Katalysatoren in verschiedenen Zweiphasengemischen umfassend eine obere Phase mit freiem tertiären Amin und eine untere Phase mit polarem Lösungsmittel und Ameisensäure/Amin-Addukt untersucht. Hierzu wurden bei den Versuchen C-1 bis C-26 jeweils 5 g des Ameisensäure-Trihexylamin-Addukts (hergestellt aus Ameisensäure und Trihexylamin im Molverhältnis Amin zu Ameisensäure von 1 : 2) bei Raumtemperatur unter Rühren in 5 g des polaren Lösungsmittels gelöst und mit 5 g Trihexylamin und 10 mg des homogenen Ru-Katalysators versetzt. Das erhaltene Zweiphasensystem wurde 30 Minuten bei Raumtemperatur kräftig gerührt. Bei den Versuchen C-27 bis C-34 wurden jeweils 2,5 g Ameisensäure mit 20 g des entsprechenden Amins gemischt und 30 Minuten bei Raumtemperatur gerührt. Nach der Reaktion wurden 5 g der Unterphase abgetrennt beziehungsweise bei einphasigen Gemischen das Reaktionsgemisch eingesetzt und zu einem Gemisch aus 10 mg [Ru $(PnBu_3)_4(H)_2$], 5 g des entsprechenden Amins und 5 g Ethylenglykol gegeben und das erhaltene Zweiphasensystem 10 Minuten bei Raumtemperatur kräftig gerührt. Anschließend wurde bei den Versuchen C-1 bis C-34 nach Trennung beider Phasen der Verteilungskoeffizient K des Rutheniums

$$K = [\text{Ru-Konzentration in oberer Phase}] / [\text{Ru-Konzentration in unterer Phase}]$$

mittels Atomadsorptionsspektrometrie (AAS) bestimmt.

Die Parameter und Ergebnisse der einzelnen Versuche sind in Tabelle 3.1 bis 3.2 wiedergegeben.

Die Beispiele zeigen, dass bei allen getesteten Systemen der Verteilungskoeffizient K des Rutheniums deutlich oberhalb von 1, bei manchen Systemen sogar deutlich oberhalb von 10 liegt. Die verschiedenen homogenen Ru-Katalysatoren waren in allen getesteten Systemen (verschiedene polare Lösungsmittel, verschiedene tertiäre Amine) jeweils in der oberen, Amin-haltigen Phase angereichert.

Beispiele D-1 bis D-2 (Hydrierung, Phasentrennung, Extraktion und Katalysatorrückführung)

Zur Untersuchung der kontinuierlichen Hydrierung, Phasentrennung, Extraktion und Katalysatorrückführung wurde eine Laboranlage wie in Fig. 3 dargestellt verwendet. Darin haben die einzelnen Buchstaben folgende Bedeutung:

A = Hydrierreaktor (270 mL Autoklav aus Hastelloy C mit Blattrührer)
B = Phasenabscheider
E = Extraktionsgefäß (350 mL Glasrührbehälter mit Glasblattrührer) .
F = Phasenabscheider
K = Lösungsmittelpumpe
L = Entspannungsventil
M = Rückführpumpe für tertiäres Amin (I) und homogenen Katalysator
N = Produktstrompumpe
O = Aminpumpe
P = Rückführpumpe für tertiäres Amin (I) und extrahierten homogenen Katalysator

Des Weiteren bedeuten:

Solvent = Lösungsmittel
Off-gas = Abgas
Amine (I) = tertiäres Amin (I)

Unter Inertbedingungen wurde unter Rühren eine Emulsion durch Lösen des homogenen Katalysators in tertiärem Amin (I) und nachfolgender Zugabe des polaren Lösungsmittels hergestellt. Die Parameter der einzelnen Versuche sind in Tabelle 4.1 und 4.2 wiedergegeben. Mit der hergestellten Emulsion wurde dann der Hydrierreaktor "A" und der Phasenabscheider "B" befüllt. Das Extraktionsgefäß "E" und der Phasenabscheider "F" wurden mit einem Gemisch aus tertiärem Amin (I) und polarem Lösungsmittel befüllt. Der Hydrierreaktor "A" wurde nun unter Rühren aufgewärmt und nach Einstellung eines $H_2$-Vordrucks mit $CO_2$ aufgepresst. Anschließend wurde weiteres $H_2$ aufgepresst und der Hydrierreaktor "A" unter den eingestellten Bedingungen für die genannte Anfangs-Reaktionszeit bei geschlossenem Entspannungsventil "L" belassen. Anschließend wurde das Entspannungsventil "L" geöffnet und die Anlage kontinuierlich betrieben. Dabei wurden als Strom 7a polares Lösungsmittel, als Strom 1 $CO_2$ und als Strom 2 $H_2$ kontinuierlich zugeführt. Über das geöffnete Entspannungsventil "L" gelangte Produktgemisch in den Phasenabscheider "B" und wurde in zwei Phasen getrennt. Die obere Phase wurde als Strom 4 wieder zum Hydrierreaktor "A" rückgeführt. Die untere Phase wurde als Strom 5 in das Extraktionsgefäß "E" gefahren. In diesem wurde zur Extraktion des restlichen homogenen Katalysators frisches tertiäres Amin (I) als Strom 10a zugegeben. Dabei wurde jeweils soviel tertiäres Amin (I) zugegeben,

wie über Strom 8a abgeführt wurde. Im nachfolgenden Phasenabscheider "F" wurde der Austrag aus dem Extraktionsgefäß "E" in zwei Phasen getrennt. Die obere Phase wurde als Strom 12 wieder zum Hydrierreaktor "A" rückgeführt. Die untere Phase wurde als Strom 8a ausgeschleust. Im Phasenabscheider "B" und Extraktionsgefäß "E" angesammeltes Gas wurde als Abgas entfernt. Die Menge an gebildeter Ameisensäure wurde aus Strom 8a durch Titration mit 0,1 N KOH in 2-Propanol gegen Phenolphthalein bestimmt. Daraus wurden die TON, die TOF und die Reaktionsrate berechnet. Die Gehalte an Ruthenium wurden mittels Atomadsorptionsspektrometrie (AAS) bestimmt. Unter diesen Bedingungen wurde die Anlage jeweils für mehrere Stunden betrieben. Die Ergebnisse der einzelnen Versuche sind ebenfalls in Tabelle 4.2 wiedergegeben.

Die Beispiele D-1 und D-2 zeigen, dass auch beim kontinuierlichen Betrieb der Anlage über die Phasentrennung auch eine sehr gute Abtrennung des homogenen Katalysators mit nachfolgender Rückführung zum Hydrierreaktor möglich ist. So enthielt die obere, rückzuführenden Phase des Phasenabscheiders "B" jeweils mit 175 Gew.-ppm (D-1) beziehungsweise 390 Gew.-ppm (D-2) eine sehr deutliche Anreicherung des homogenen Katalysators gegenüber nur 15 Gew.-ppm (D-1) beziehungsweise 26 Gew.-ppm (D-2) in der unteren Phase (Produktstrom). Durch die bevorzugte Extraktion mit tertiärem Amin (I) im Extraktionsgefäß "E" mit nachfolgender Phasentrennung im Phasenabscheider "F" konnte der Produktstrom an homogenen Katalysator weiter abgereichert werden, im Beispiel D-1 von 15 Gew.-ppm auf 10 Gew.-ppm sowie im Beispiel D-2 von 26 Gew.-ppm auf 11 Gew.-ppm, was einer weiteren Abreicherung auf 67% beziehungsweise 42% entspricht. Die obere Phase im Phasenabscheider "F" enthielt 46 Gew.-ppm (D-1) beziehungsweise 160 Gew.-ppm (D-2), die jeweils zum Hydrierreaktor rückgeführt werden konnten. Somit ist es möglich, einen höheren Anteil des homogenen Katalysators direkt abzutrennen und in der Hydrierung erneut einzusetzen.

Tabelle 1.1

| | Beispiel A-1 | Beispiel A-2 | Beispiel A-3 | Beispiel A-4 |
|---|---|---|---|---|
| Tertiäres Amin | 49 g Trihexylamin | 50 g Trihexylamin | 30 g Trihexylamin | 50 g Trihexylamin |
| Polares Lösungsmittel | 20 g 1,4-Butandiol | 25 g Ethylenglykoldiformiat 25 g Ethylenglykol | 70 g 2-Methyl-1,3-propandiol | 50 g 2-Methyl-1,3-propandiol 5,0 g $C_{17}$-Alkanolgemisch |
| Katalysator | 0,1 g $[Ru(P^nBu_3)_4(H)_2]$ | 0,1 g $[Ru(P^nBu_3)_4(H)_2]$ | 0,1 g $[Ru(P^nBu_3)_4(H)_2]$ | 0,1 g $[Ru(P^nBu_3)_4(H)_2]$ |
| Besonderheit | Zweimaliges Spülen mit 1 MPa abs $H_2$ | --- | --- | --- |
| Aufpressen von $CO_2$ | Mit 14,4 g auf 2,9 MPa abs | Mit 17,6 g auf 2,7 MPa abs | Mit 15,8 g auf 2,6 MPa abs | Mit 15,7 g auf 3,0 MPa abs |
| Aufpressen von $H_2$ | Auf 8,9 MPa abs | Auf 9,0 MPa abs | Auf 8,7 MPa abs | Auf 9,1 MPa abs |
| Erwärmen | Auf 50°C | Auf 50°C | Auf 50°C | Auf 50°C |
| Druckänderung | Auf 9,4 MPa abs | Auf 9,9 MPa abs | Auf 8,7 MPa abs | Auf 9,0 MPa abs |
| Reaktionszeit | 5 Stunden | 1 Stunde | 1 Stunde | 1 Stunde |
| Besonderheit | --- | --- | --- | $C_{17}$-Alkanolgemisch befand sich in der Amin/Katalysator-Phase |
| Verteilungskoeffizient K des Rutheniums | 28,8 | nicht bestimmt | nicht bestimmt | nicht bestimmt |
| TON | 321 | 150 | 727 | 539 |
| TOF | 64 $h^{-1}$ | 150 $h^{-1}$ | 727 $h^{-1}$ | 539 $h^{-1}$ |
| Reaktionsrate | 0,06 mol $kg^{-1}$ $h^{-1}$ | 0,17 mol $kg^{-1}$ $h^{-1}$ | 0,90 mol $kg^{-1}$ $h^{-1}$ | 0,60 mol $kg^{-1}$ $h^{-1}$ |

Tabelle 1.2

| | Beispiel A-5 | Beispiel A-6 | Beispiel A-7 | Beispiel A-8 |
|---|---|---|---|---|
| Tertiäres Amin | 50 g Trihexylamin | 50 g Trihexylamin | 30 g Trihexylamin | 50 g Trihexylamin |
| Polares Lösungsmittel | 50 g 2-Methyl-1,3-propandiol | 50 g 2-Methyl-1,3-propandiol | 70 g 2-Methyl-1,3-propandiol | 50 g Ethylenglykoldiformiat 5,0 g 2-Octyl-dodecanol |
| Katalysator | 0,1 g $[Ru(P^nBu_3)_4(H)_2]$ | 0,4 g $[Ru(P^nBu_3)_4(H)_2]$ | 0,4 g $[Ru(P^nBu_3)_4(H)_2]$ 0,2 g $P^nBu_3$ | 0,1 g $[Ru(P^nBu_3)_4(H)_2]$ |
| Besonderheit | --- | --- | Zusatz von 1,0 g Wasser | --- |
| Aufpressen von $CO_2$ | Mit 14,0 g auf 2,8 MPa abs | Mit 16,2 g auf 2,9 MPa abs | Mit 20,1 g auf 3,8 MPa abs | Mit 14,3 g auf 2,6 MPa abs |
| Aufpressen von $H_2$ | Auf 10,8 MPa abs | Auf 7,1 MPa abs | Auf 8,1 MPa abs | Auf 7,3 MPa abs |
| Erwärmen | Auf 50°C | Auf 50°C | Auf 50°C | Auf 50°C |
| Druckänderung | Auf 11,3 MPa abs | Auf 8,2 MPa abs | Auf 8,9 MPa abs | Auf 8,4 MPa abs |
| Reaktionszeit | 1 Stunde | 1 Stunde | 1 Stunde | 1 Stunde |
| Besonderheit | --- | --- | --- | --- |
| Verteilungskoeffizient K des Rutheniums | nicht bestimmt | nicht bestimmt | nicht bestimmt | nicht bestimmt |
| TON | 304 | 171 | 166 | 157 |
| TOF | 304 $h^{-1}$ | 171 $h^{-1}$ | 166 $h^{-1}$ | 157 $h^{-1}$ |
| Reaktionsrate | 0,38 mol $kg^{-1}$ $h^{-1}$ | 0,83 mol $kg^{-1}$ $h^{-1}$ | 0,75 mol $kg^{-1}$ $h^{-1}$ | 0,17 mol $kg^{-1}$ $h^{-1}$ |

Tabelle 1.3

| | Beispiel A-9 | Beispiel A-10 | Beispiel A-11 | Beispiel A-12 |
|---|---|---|---|---|
| Tertiäres Amin | 50 g Trihexylamin | 50 g Trihexylamin | 50 g N,N-Dimethyldecylamin | 50 g Tributylamin |
| Polares Lösungsmittel | 50 g Ethylenglykol | 50 g 2-Methyl-1,3-propandiol | 50 g Ethylenglykol | 50 g 2-Methyl-1,3-propandiol |
| Katalysator | 0,1 g $[Ru(P^nBu_3)_4(H)_2]$ | 0,1 g $[Ru(P^nBu_3)_4(H)_2]$ | 0,1 g $[Ru(P^nBu_3)_4(H)_2]$ | 0,2 g $[Ru(P^nBu_3)_4(H)_2]$ |
| Besonderheit | --- | Zusatz von 1,0 g Wasser | --- | --- |
| Aufpressen von $CO_2$ | Mit 16,8 g auf 3,3 MPa abs | Mit 16,0 g auf 3,1 MPa abs | Mit 13,3 g auf 2,2 MPa abs | Mit 18,2 g auf 2,9 MPa abs |
| Aufpressen von $H_2$ | Auf 8,1 MPa abs | Auf 8,1 MPa abs | Auf 7,2 MPa abs | Auf 7,9 MPa abs |
| Erwärmen | Auf 50°C | Auf 50°C | Auf 50°C | Auf 50°C |
| Druckänderung | Auf 7,5 MPa abs | Auf 8,0 MPa abs | Auf 8,5 MPa abs | Auf 8,3 MPa abs |
| Reaktionszeit | 1 Stunde | 1 Stunde | 1 Stunde | 1 Stunde |
| Besonderheit | --- | --- | --- | --- |
| Verteilungskoeffizient K des Rutheniums | 225 | nicht bestimmt | nicht bestimmt | nicht bestimmt |

(fortgesetzt)

| | Beispiel A-9 | Beispiel A-10 | Beispiel A-11 | Beispiel A-12 |
|---|---|---|---|---|
| TON | 374 | 444 | 271 | 199 |
| TOF | 374 $h^{-1}$ | 444 $h^{-1}$ | 271 $h^{-1}$ | 199 $h^{-1}$ |
| Reaktionsrate | 0,44 mol $kg^{-1}$ $h^{-1}$ | 0,56 mol $kg^{-1}$ $h^{-1}$ | 0,31 mol $kg^{-1}$ $h^{-1}$ | 0,52 mol $kg^{-1}$ $h^{-1}$ |

Tabelle 1.4

| | Beispiel A-13 | Beispiel A-14 | Beispiel A-15 | Beispiel A-16 |
|---|---|---|---|---|
| Tertiäres Amin | 50 g N-Methyl-dicyclohexylamin | 50 g Trioctylamin | 50 g Tripropylamin | 50 g N,N-Dimethyl-cyclohexylamin |
| Polares Lösungsmittel | 50 g 2-Methyl-1,3-propandiol | 50 g 2-Methyl-1,3-propandiol | 50 g 2-Methyl-1,3-propandiol | 50 g Ethylenglykol |
| Katalysator | 0,1 g $[Ru(P^nBu_3)_4(H)_2]$ | 0,2 g $[Ru(P^nBu_3)_4(H)_2]$ | 0,2 g $[Ru(P^nBu_3)_4(H)_2]$ | 0,1 g $[Ru(P^nBu_3)_4(H)_2]$ |
| Besonderheit | --- | --- | --- | --- |
| Aufpressen von $CO_2$ | Mit 15,0 g auf 3,1 MPa abs | Mit 15,7 g auf 3,2 MPa abs | Mit 15,0 g auf 2,0 MPa abs | Mit 15,0 g auf 2,3 MPa abs |
| Aufpressen von $H_2$ | Auf 8,1 MPa abs | Auf 8,2 MPa abs | Auf 7,1 MPa abs | Auf 8,1 MPa abs |
| Erwärmen | Auf 50°C | Auf 50°C | Auf 50°C | Auf 50°C |
| Druckänderung | Auf 7,30 MPa abs | Auf 8,8 MPa abs | Auf 7,3 MPa abs | Auf 8,2 MPa abs |
| Reaktionszeit | 1 Stunde | 1 Stunde | 1 Stunde | 1 Stunde |
| Besonderheit | --- | --- | --- | --- |
| Verteilungskoeffizient K des Rutheniums | nicht bestimmt | nicht bestimmt | nicht bestimmt | nicht bestimmt |
| TON | 404 | 171 | 162 | 325 |
| TOF | 404 $h^{-1}$ | 171 $h^{-1}$ | 162 $h^{-1}$ | 325 $h^{-1}$ |
| Reaktionsrate | 0,49 mol $kg^{-1}$ $h^{-1}$ | 0,41 mol $kg^{-1}$ $h^{-1}$ | 0,38 mol $kg^{-1}$ $h^{-1}$ | 0,42 mol $kg^{-1}$ $h^{-1}$ |

Tabelle 1.5

| | Beispiel A-17 | Beispiel A-18 | Beispiel A-19 | Beispiel A-20 |
|---|---|---|---|---|
| Tertiäres Amin | 50 g Tributylamin | 50 g N-Methyl-dicyclohexylamin | 50 g Trihexylamin | 50 g Trihexylamin |
| Polares Lösungsmittel | 50 g 1,4-Butandiol | 50 g 1,4-Butandiol | 50 g 2-Methyl-1,3-propandiol | 50 g 2-Methyl-1,3-propandiol |
| Katalysator | 0,2 g $[Ru(P^nBu_3)_4(H)_2]$ | 0,2 g $[Ru(P^nBu_3)_4(H)_2]$ | 0,15 g $[Rh(PPh_3)_4(H)]$ | 0,3 g $[Ru(P^nOctyl_3)_4(H)_2]$ |
| Besonderheit | --- | --- | --- | --- |
| Aufpressen von $CO_2$ | Mit 15,8 g auf 2,7 MPa abs | Mit 14,9 g auf 2,9 MPa abs | Mit 15,8 g auf 2,9 MPa abs | Mit 15,9 g auf 3,3 MPa abs |
| Aufpressen von $H_2$ | Auf 7,7 MPa abs | Auf 7,9 MPa abs | Auf 7,9 MPa abs | Auf 9,3 MPa abs |
| Erwärmen | Auf 50°C | Auf 50°C | Auf 50°C | Auf 50°C |
| Druckänderung | Auf 8,0 MPa abs | Auf 7,8 MPa abs | Auf 8,6 MPa abs | Auf 9,5 MPa abs |
| Reaktionszeit | 1 Stunde | 1 Stunde | 5 Stunden | 1 Stunde |
| Besonderheit | --- | --- | --- | --- |
| Verteilungskoeffizient K des Rutheniums | nicht bestimmt | nicht bestimmt | nicht bestimmt | nicht bestimmt |
| TON | 55 | 250 | 38 | 150 |
| TOF | 55 $h^{-1}$ | 250 $h^{-1}$ | 7,6 $h^{-1}$ | 150 $h^{-1}$ |
| Reaktionsrate | 0,16 mol $kg^{-1}$ $h^{-1}$ | 0,58 mol $kg^{-1}$ $h^{-1}$ | 0,05 mol $kg^{-1}$ $h^{-1}$ | 0,29 mol $kg^{-1}$ $h^{-1}$ |

Tabelle 1.6

| | Beispiel A-21 | Beispiel A-22 | Beispiel A-23 | Beispiel A-24 |
|---|---|---|---|---|
| Tertiäres Amin | 50 g Trihexylamin | 50 g Trihexylamin | 50 g Trihexylamin | 50 g Trihexylamin |
| Polares Lösungsmittel | 50 g 2-Methyl-1,3-propandiol<br>3,0 g $C_{17}$-Alkanolgemisch | 50 g 2-Methyl-1,3-propandiol<br>3,0 g $C_{17}$-Alkanolgemisch | 50 g 2-Methyl-1,3-propandiol<br>2,0 g 2-Ethylhexansäure | 50 g 2-Methyl-1,3-propandiol |
| Katalysator | 0,1 g $[Ru(P^nBu_3)_4(H)_2]$ | 0,1 g $[Ru(P^nBu_3)_4(H)_2]$<br>0,09 g Bis(dicyclohexyl)-phosphinoethan | 0,1 g $[Ru(P^nBu_3)_4(H)_2]$<br>0,09 g Bis(dicyclohexyl)-phosphinoethan | 0,1 g $[Ru(PPh_3)_3(Cl)(OAc)]$<br>0,08 g $P^nBu_3$ |
| Besonderheit | --- | --- | --- | --- |
| Aufpressen von $CO_2$ | Mit 14,7 g auf 2,9 MPa abs | Mit 15,0 g auf 3,0 MPa abs | Mit 15,4 g auf 2,8 MPa abs | Mit 15,2 g auf 3,0 MPa abs |
| Aufpressen von $H_2$ | Auf 10,9 MPa abs | Auf 11,0 MPa abs | Auf 10,8 MPa abs | Auf 8,0 MPa abs |
| Erwärmen | Auf 50°C | Auf 50°C | Auf 50°C | Auf 50°C |
| Druckänderung | Auf 11,6 MPa abs | Auf 11,4 MPa abs | Auf 11,6 MPa abs | Auf 8,3 MPa abs |
| Reaktionszeit | 1 Stunde | 1 Stunde | 1 Stunde | 1 Stunde |
| Besonderheit | $C_{17}$-Alkanolgemisch befand sich in der Amin/Katalysator-Phase | $C_{17}$-Alkanolgemisch befand sich in der Amin/Katalysator-Phase | 2-Ethylhexansäure befand sich in der Amin/Katalysator-Phase | --- |
| Verteilungskoeffizient K des Rutheniums | 4,8 | 3,0 | 4,8 | 5,7 |
| TON | 497 | 956 | 838 | 353 |
| TOF | 497 $h^{-1}$ | 956 $h^{-1}$ | 838 $h^{-1}$ | 353 $h^{-1}$ |
| Reaktionsrate | 0,55 mol $kg^{-1}$ $h^{-1}$ | 1,08 mol $kg^{-1}$ $h^{-1}$ | 0,93 mol $kg^{-1}$ $h^{-1}$ | 0,36 mol $kg^{-1}$ $h^{-1}$ |

Tabelle 1.7

| | Beispiel A-25 | Beispiel A-26 | Beispiel A-27 |
|---|---|---|---|
| Tertiäres Amin | 50 g N-Methyldicyclohexylamin | 50 g Tributylamin | 25 g Trihexylamin |
| Polares Lösungsmittel | 50 g 2-Methyl-1,3-propandiol | 50 g 2-Methyl-1,3-propandiol | 25 g 2-Methyl-1,3-propandiol |
| Katalysator | 0,1 g $[Ru(P^nBu_3)_4(H)_2]$ | 0,1 g $[Ru(P^nBu_3)_4(H)_2]$<br>0,09 g Bis(dicyclohexyl)-phosphinoethan | 0,05 g $[Ru(P^nBu_3)_4(H)_2]$<br>0,042 g Bis(dicyclohexyl)-phosphinoethan |
| Besonderheit | --- | --- | 100 mL Autoklav mit Blattrührer |
| Aufpressen von $CO_2$ | Mit 15,8 g auf 3,7 MPa abs | Mit 15,9 g auf 2,65 MPa abs | Mit 7,9 g auf 2,6 MPa abs |
| Aufpressen von $H_2$ | Auf 8,7 MPa abs | Auf 7,7 MPa abs | Auf 8,1 MPa abs |
| Erwärmen | Auf 50°C | Auf 50°C | Auf 50°C |
| Druckänderung | Auf 7,6 MPa abs | Auf 8,0 MPa abs | nicht bestimmt |
| Reaktionszeit | 1 Stunde | 1 Stunde | 1 Stunde |
| Besonderheit | --- | --- | --- |
| Verteilungskoeffizient K des Rutheniums | 15,3 | 10,6 | 5,6 |
| TON | 841 | 792 | 430 |
| TOF | 841 $h^{-1}$ | 792 $h^{-1}$ | 430 $h^{-1}$ |
| Reaktionsrate | 0,97 mol $kg^{-1}$ $h^{-1}$ | 0,85 mol $kg^{-1}$ $h^{-1}$ | 0,56 mol $kg^{-1}$ $h^{-1}$ |

Tabelle 2.1

| | Beispiel B-1 | Beispiel B-2 | Beispiel B-3 | Beispiel B-4 |
|---|---|---|---|---|
| Tertiäres Amin | Trioctylamin | Trihexylamin | Tripentylamin | N-Methyl-dicyclohexylamin |
| Polares Lösungsmittel | 2-Methyl-1,3-propandiol | 2-Methyl-1,3-propandiol | 2-Methyl-1,3-propandiol | 2-Methyl-1,3-propandiol |
| Zahl der Flüssigphasen vor $CO_2$-Aufpressung | Zwei | Zwei | Zwei | Zwei |
| Verhalten der Amin haltigen Flüssigphase (B) | Volumenzunahme (Einlösung von $CO_2$) | Volumenzunahme (Einlösung von $CO_2$) | Aminphase löst sich zum größten Teil in $CO_2$-Phase ein | Volumenzunahme (Einlösung von $CO_2$) |
| Verhalten der Lösungsmittel und Addukt haltigen Flüssigphase (A) | Keine sichtbare Veränderung | Keine sichtbare Veränderung | Keine sichtbare Veränderung | Keine sichtbare Veränderung |
| Besonderheit | --- | --- | --- | --- |

Tabelle 2.2

| | Beispiel B-5 | Beispiel B-6 | Beispiel B-7 | Beispiel B-8 |
|---|---|---|---|---|
| Tertiäres Amin | Trihexylamin | N,N-Dimethyldecylamin | N, N-Dimethylcyclohexylamin | N,N-Dioctylmethylamin |
| Polares Lösungsmittel | 1,4-Butandiol | 1,4-Butandiol | 1,4-Butandiol | 2-Methyl-1,3-propandiol |
| Zahl der Flüssigphasen vor $CO_2$-Aufpressung | Zwei | Zwei | Zwei | Zwei |
| Verhalten der Amin haltigen Flüssigphase (B) | Volumenzunahme (Einlösung von $CO_2$) | Volumenzunahme (Einlösung von $CO_2$) | Volumenzunahme (Einlösung von $CO_2$) | Volumenzunahme (Einlösung von $CO_2$) |
| Verhalten der Lösungsmittel und Addukt haltigen Flüssigphase (A) | Keine sichtbare Veränderung | Keine sichtbare Veränderung | Keine sichtbare Veränderung | Keine sichtbare Veränderung |
| Besonderheit | --- | --- | --- | --- |

Tabelle 2.3

| | Beispiel B-9 | Beispiel B-10 | Beispiel B-11 | Beispiel B-12 |
|---|---|---|---|---|
| Tertiäres Amin | N,N-Dimethylnonylamin | N-Ethyldiisopropylamin | Tris(2-ethyl-hexyl)amin | N-Ethyldiisopropylamin |
| Polares Lösungsmittel | 2-Methyl-1,3-propandiol | 1,4-Butandiol | 1,4-Butandiol | 2-Methyl-1,3-propandiol |
| Zahl der Flüssigphasen vor $CO_2$-Aufpressung | Zwei | Zwei | Zwei | Zwei |
| Verhalten der Amin haltigen Flüssigphase (B) | Volumenzunahme (Einlösung von $CO_2$) | Volumenzunahme (Einlösung von $CO_2$) | Aminphase löst sich zum größten Teil in $CO_2$-Phase ein | Volumenzunahme (Einlösung von $CO_2$) |
| Verhalten der Lösungsmittel und Addukt haltigen Flüssigphase (A) | Keine sichtbare Veränderung | Keine sichtbare Veränderung | Keine sichtbare Veränderung | Keine sichtbare Veränderung |
| Besonderheit | --- | --- | --- | --- |

Tabelle 3.1

| Beispiel | Tertiäres Amin | Polares Lösungsmittel | Faktor x im eingestzten Ameisensäure/Amin-Addukt Amin · x HCOOH | Katalysator | Verteilungskoeffizient K des Rutheniums |
|---|---|---|---|---|---|
| C-1 | Trihexylamin | Sulfolan | 2,0 | $[Ru(P^nBu_3)_4(H)_2]$ | 7,8 |
| C-2 | Trihexylamin | Sulfolan | 2,0 | $[Ru(P^nOctyl_3)_4(H)_2]$ | 35 |
| C-3 | Trihexylamin | Dimethylsulfoxid (DMSO) | 2,0 | $[Ru(P^nBu_3)_4(H)_2]$ | 3,4 |
| C-4 | Trihexylamin | Dimethylsulfoxid (DMSO) | 2,0 | $[Ru(P^nOctyl_3)_4(H)_2]$ | 25,7 |
| C-5 | Trihexylamin | Dimethylformamid | 2,0 | $[Ru(P^nBu_3)_4(H)_2]$ | 6,2 |
| C-6 | Trihexylamin | Dimethylformamid | 2,0 | $[Ru(P^nOctyl_3)_4(H)_2]$ | 4,0 |
| C-7 | Trihexylamin | Ethylenglykol | 2,0 | $[Ru(P^nBu_3)_4(H)_2]$ | 16,3 |
| C-8 | Trihexylamin | Ethylenglykol | 2,0 | $[Ru(P^nOctyl_3)_4(H)_2]$ | 31,3 |
| C-9 | Trihexylamin | 1,3-Propandiol | 2,0 | $[Ru(P^nBu_3)_4(H)_2]$ | 8,9 |
| C-10 | Trihexylamin | 1,3-Propandiol | 2,0 | $[Ru(P^nOctyl_3)_4(H)_2]$ | 2.3 |
| C-11 | Trihexylamin | 1,4-Butandiol | 2,0 | $[Ru(P^nBu_3)_4(H)_2]$ | 18,5 |
| C-12 | Trihexylamin | 1,4-Butandiol | 2,0 | $[Ru(P^nOctyl_3)_4(H)_2]$ | 43,3 |
| C-13 | Trihexylamin | Diethylenglykol | 2,0 | $[Ru(P^nBu_3)_4(H)_2]$ | 16,0 |
| C-14 | Trihexylamin | Diethylenglykol | 2,0 | $[Ru(P^nOctyl_3)_4(H)_2]$ | 26,7 |
| C-15 | Trihexylamin | 1,5-Pentandiol | 2,0 | $[Ru(P^nBu_3)_4(H)_2]$ | 4,3 |
| C-16 | Trihexylamin | 1,5-Pentandiol | 2,0 | $[Ru(P^nOctyl_3)_4(H)_2]$ | 19,4 |
| C-17 | Trihexylamin | Dipropylenglykol | 2,0 | $[Ru(P^nBu_3)_4(H)_2]$ | 4,6 |
| C-18 | Trihexylamin | Dipropylenglykol | 2,0 | $[Ru(P^nOctyl_3)_4(H)_2]$ | 2,6 |
| C-19 | Trihexylamin | 2-Methyl-1,3-propandiol | 2,0 | $[Ru(P^nBu_3)_4(H)_2]$ | 20,0 |
| C-20 | Trihexylamin | 2-Methyl-1,3-propandiol | 2,0 | $[Ru(P^nOctyl_3)_4(H)_2]$ | 24,0 |

Tabelle 3.2

| Beispiel | Tertiäres Amin | Polares Lösungsmittel | Faktor x im Ameisensäure/Amin-Addukt Amin · x HCOOH | Katalysator | Verteilungskoeffizient K des Rutheniums |
|---|---|---|---|---|---|
| C-21 | Trihexylamin | Ethylenglykoldiformiat | 2,0 | $[Ru(P^nBu_3)_4(H)_2]$ | 28,1 |
| C-22 | Trihexylamin | Ethylenglykoldiformiat | 2,0 | $[Ru(P^nOctyl_3)_4(H)_2]$ | 42,5 |
| C-23 | Trihexylamin | 1,4-Butylenglykoldiformiat | 2,0 | $[Ru(P^nBu_3)_4(H)_2]$ | 3,8 |
| C-24 | Trihexylamin | 1,4-Butylenglykoldiformiat | 2,0 | $[Ru(P^nOctyl_3)_4(H)_2]$ | 10,8 |
| C-25 | Trihexylamin | 2-Methyl-1,3-propylenglykoldiformiat | 2,0 | $[Ru(P^nBu_3)_4(H)_2]$ | 4,7 |
| C-26 | Trihexylamin | 2-Methyl-1,3-propylenglykoldiformiat | 2,0 | $[Ru(P^nOctyl_3)_4(H)_2]$ | 8,5 |
| C-27 | N, N-Dimethyldecylamin | Ethylenglykol | nicht bestimmt | $[Ru(P^nBu_3)_4(H)_2]$ | 8,9 |
| C-28 | Trioctylamin | Ethylenglykol | nicht bestimmt | $[Ru(P^nBu_3)_4(H)_2]$ | 1,5 |
| C-29 | Tripentylamin | Ethylenglykol | nicht bestimmt | $[Ru(P^nBu_3)_4(H)_2]$ | 9,5 |
| C-30 | N-Methydicyclohexylamin | Ethylenglykol | nicht bestimmt | $[Ru(P^nBu_3)_4(H)_2]$ | 18,7 |
| C-31 | N,N-Dimethyl-cyclohexylamin | Ethylenglykol | nicht bestimmt | $[Ru(P^nBu_3)_4(H)_2]$ | 20,4 |
| C-32 | N-Ethyl-diisopropylamin | Ethylenglykol | nicht bestimmt | $[Ru(P^nBu_3)_4(H)_2]$ | 27,0 |
| C-33 | Tripropylamin | Ethylenglykol | nicht bestimmt | $[Ru(P^nBu_3)_4(H)_2]$ | 50,0 |
| C-34 | Tributylamin | Ethylenglykol | nicht bestimmt | $[Ru(P^nBu_3)_4(H)_2]$ | 160 |

Tabelle 4.1

| | | Beispiel D-1 | Beispiel D-2 |
|---|---|---|---|
| Emulsion für Hydrierreaktor "A" und Phasenabscheider "B" | Tertiäres Amin | 73 g Trihexylamin | 73 g Trihexylamin |
| | Polares Lösungsmittel | 93 g 2-Methyl-1,3-propandiol | 93 g 2-Methyl-1,3-propandiol |
| | Katalysator | 0,37 g $[Ru(P^nBu_3)_4(H)_2]$ 0,34 g Bis(dicyclohexyl)-phosphinoethan | 0,74 g $[Ru(P^nBu_3)_4(H)_2]$ 0,34 g $P^nBu_3$ |
| Gemisch für Extraktionsgefäß "E" und Phasenabscheider "F" | Tertiäres Amin | 50 Gew.-% Trihexylamin | 33 Gew.-% Trihexylamin |
| | Polares Lösungsmittel | 50 Gew.-% 2-Methyl-1,3-propandiol | 67 Gew.-% 2-Methyl-1,3-propandiol |
| Anfahren | Erwärmung | Unter Rühren (1000 U/min) auf 50°C | Unter Rühren (1300 U/min) auf 50°C |
| | $H_2$-Vordruck | 3,0 MPa abs | 3,6 MPa abs |
| | Aufpressen von $CO_2$ | Mit 12,0 g $CO_2$ auf 7,4 MPa abs | Mit 13,0 g $CO_2$ auf 7,4 MPa abs |
| | Aufpressen von $H_2$ | Auf 8,1 MPa abs | Auf 8,9 MPa abs |
| | Anfangs-Reaktionszeit | 1 Stunde | 1 Stunde |

Tabelle 4.2

| | | Beispiel D-1 | Beispiel D-2 |
|---|---|---|---|
| Kontinuierlicher Betrieb | $CO_2$ | 21,8 g/h | 13,0 g/h |
| | $H_2$ | 2,7 g/h | 2,7 g/h |
| | Polares Lösungsmittel | 83 g/h | 43 g/h |
| | Hydrierreaktor "A" | 50°C, 8,0MPa abs | 50°C, 8,8 MPa abs |
| | Phasenabscheider "B" | 50°C, 0,1 MPa abs | 50°C, 0,1 MPa abs |
| | Extraktionsgefäß "E" | 20°C, 0,1 MPa abs | 20°C, 0,1 MPa abs |
| | Phasenabscheider "F" | 20°C, 0,1 MPa abs | 20°C, 0,1 MPa abs |
| Ameisensäure | TON | 744 | 194 |
| | TOF | 149 $h^{-1}$ | 56 $h^{-1}$ |
| | Reaktionsrate | 0,43 mol $kg^{-1}$ $h^{-1}$ | 0,32 mol $kg^{-1}$ $h^{-1}$ |
| Ruthenium-Gehalte | Untere Phase in Phasenabscheider "B" | 15 Gew.-ppm | 26 Gew.-ppm |
| | Obere Phase in Phasenabscheider "B" | 175 Gew.-ppm | 390 Gew.-ppm |
| | Untere Phase in Phasenabscheider "F" | 10 Gew.-ppm | 11 Gew.-ppm |
| | Obere Phase in Phasenabscheider "F" | 46 Gew.-ppm | 160 Gew.-ppm |

**Patentansprüche**

1. Verfahren zur Herstellung von Ameisensäure durch Hydrierung von Kohlendioxid in Gegenwart eines Katalysators enthaltend ein Element aus der 8., 9. oder 10. Gruppe des Periodensystems, eines tertiären Amins (I) und eines polaren Lösungsmittels (III) bei einem Druck von 0,2 bis 30 MPa abs und einer Temperatur von 20 bis 200°C unter Ausbildung zweier Flüssigphasen, Trennung der einen, mit dem Ameisensäure/Amin-Addukt (II) angereicherten

Flüssigphase (A) von der anderen Flüssigphase (B) und Rückführung der Flüssigphase (B) zum Hydrierreaktor, **dadurch gekennzeichnet, dass** man als Katalysator einen homogenen Katalysator einsetzt und dieser zusammen mit dem tertiären Amin (I) in der Flüssigphase (B) angereichert vorliegt;

(a) als tertiäres Amin (I) ein Amin einsetzt, welches bei einem Druck von 1013 hPa abs einen um mindestens 5°C höheren Siedepunkt als Ameisensäure aufweist und in der Flüssigphase (B) angereichert vorliegt;
(b) als polares Lösungsmittel (III) ein Lösungsmittel einsetzt, dessen elektrostatischer Faktor $\geq 200 \cdot 10^{-30}$ Cm beträgt und welches bei einem Druck von 1013 hPa abs einen um mindestens 5°C höheren Siedepunkt als Ameisensäure aufweist und in der Flüssigphase (A) angereichert vorliegt;
(c) das Ameisensäure/Amin-Addukt (II) der abgetrennten Flüssigphase (A) in einer Destillationseinheit thermisch in freie Ameisensäure und freies tertiäres Amin (I) spaltet;
(d) die freie Ameisensäure destillativ entfernt; und
(e) das im Sumpf der Destillationseinheit enthaltene freie tertiäre Amin (I) und das polare Lösungsmittel (III) zum Hydrierreaktor rückführt.

**2.** Verfahren nach Anspruch 1, **dadurch gekennzeichnet, dass** man einen homogenen Katalysator einsetzt, welcher Ruthenium enthält.

**3.** Verfahren nach den Ansprüchenl bis 2, **dadurch gekennzeichnet, dass** man als homogenen Katalysator einen metallorganischen Komplex verwendet, welcher ein Element aus der 8., 9. oder 10. Gruppe des Periodensystems und mindestens eine Phosphin-Gruppe mit mindestens einem unverzweigten oder verzweigten, acyclischen oder cyclischen, aliphatischen Rest mit 1 bis 12 Kohlenstoffatomen enthält, wobei einzelne Kohlenstoffatome auch durch >P- substituiert sein können.

**4.** Verfahren nach den Ansprüchen 1 bis 3, **dadurch gekennzeichnet, dass** man bei Merkmal (e) den Sumpf der Destillationseinheit in eine das freie tertiäre Amin (I) enthaltende Phase und eine das polare Lösungsmittel (III) enthaltende Phase trennt und beide Phasen getrennt zum Hydrierreaktor rückführt, wobei man die das freie tertiäre Amin (I) enthaltende Phase über eine Extraktionseinheit zum Hydrierreaktor rückführt und in der genannten Extraktionseinheit homogenen Katalysator aus der abgetrennten Flüssigphase (A) extrahiert, bevor das Ameisensäure/Amin-Addukt (II) der abgetrennten Flüssigphase (A) gemäß Merkmal (c) in einer Destillationseinheit thermisch in freie Ameisensäure und freies tertiäres Amin (I) gespalten wird.

**5.** Verfahren nach den Ansprüchen 1 bis 4 **dadurch gekennzeichnet, dass** man als tertiäres Amin (I) ein Amin der allgemeinen Formel (Ia)

$NR^1R2R^3$ (Ia),

in der die Reste $R^1$ bis $R^3$ gleich oder verschieden sind und unabhängig voneinander einen unverzweigten oder verzweigten, acyclischen oder cyclischen, aliphatischen, araliphatischen oder aromatischen Rest mit jeweils 1 bis 16 Kohlenstoffatomen darstellen, wobei einzelne Kohlenstoffatome unabhängig voneinander auch durch eine Heterogruppe ausgewählt aus der Gruppe -O- und >Nsubstituiert sein können sowie zwei oder alle drei Reste unter Bildung einer mindestens jeweils vier Atome umfassenden Kette auch miteinander verbunden sein können, einsetzt.

**6.** Verfahren nach Anspruch 5, **dadurch gekennzeichnet, dass** man als tertiäres Amin (I) ein Amin der allgemeinen Formel (Ia), in der die Reste $R^1$ bis $R^3$ unabhängig voneinander ausgewählt sind aus der Gruppe $C_1$- bis $C_{12}$-Alkyl, $C_5$- bis $C_8$-Cycloalkyl, Benzyl und Phenyl, einsetzt.

**7.** Verfahren nach den Ansprüchen 5 bis 6, **dadurch gekennzeichnet, dass** man als tertiäres Amin (I) ein gesättigtes Amin der allgemeinen Formel (Ia) einsetzt.

**8.** Verfahren nach Anspruch 7, **dadurch gekennzeichnet, dass** man als tertiäres Amin (I) ein Amin der allgemeinen Formel (Ia), in der die Reste $R^1$ bis $R^3$ unabhängig voneinander ausgewählt sind aus der Gruppe $C_5$- bis $C_8$-Alkyl, einsetzt.

**9.** Verfahren nach den Ansprüchen 1 bis 8, **dadurch gekennzeichnet, dass** man als polares Lösungsmittel (III) einen aliphatischen, gesättigten Kohlenwasserstoff mit 2 bis 5 OH-Gruppen sowie deren Ameisensäureester einsetzt.

**10.** Verfahren nach den Ansprüchen 1 bis 9, **dadurch gekennzeichnet, dass** man die Hydrierung bei einem Molverhältnis von Kohlendioxid zu tertiärem Amin (I) von 0,1 bis 10 durchführt.

**11.** Verfahren nach den Ansprüchen 1 bis 10, **dadurch gekennzeichnet, dass** man die Trennung der einen, mit dem Ameisensäure/Amin-Addukt (II) und dem polaren Lösungsmittel (III) angereicherten Flüssigphase (A) von der anderen, mit dem tertiären Amin (I) angereicherten Flüssigphase (B), sowie die Rückführung der Flüssigphase (B) zum Hydrierreaktor bei einem Druck von 1 bis 30 MPa abs durchführt.

## Claims

**1.** A process for preparing formic acid by hydrogenation of carbon dioxide in the presence of a catalyst comprising an element of group 8, 9 or 10 of the Periodic Table, a tertiary amine (I) and a polar solvent (III) at a pressure of from 0.2 to 30 MPa abs and a temperature of from 20 to 200°C to form two liquid phases, separation of the one liquid phase (A) enriched with the formic acid/amine adduct (II) from the other liquid phase (B) and recirculation of the liquid phase (B) to the hydrogenation reactor, wherein a homogeneous catalyst is used as catalyst and this is present in enriched form together with the tertiary amine (I) in the liquid phase (B);

(a) an amine which at a pressure of 1013 hPa abs has a boiling point which is at least 5°C higher than that of formic acid and is present in enriched form in the liquid phase (B) is used as tertiary amine (I);
(b) a solvent whose electrostatic factor is $\geq 200 \cdot 10^{-30}$ Cm and which at a pressure of 1013 hPa abs has a boiling point which is at least 5°C higher than that of formic acid and is present in enriched form in the liquid phase (A) is used as polar solvent (III);
(c) the formic acid/amine adduct (II) of the liquid phase (A) which has been separated off is thermally dissociated into free formic acid and free tertiary amine (I) in a distillation unit;
(d) the free formic acid is removed by distillation; and
(e) the free tertiary amine (I) comprised in the bottoms from the distillation unit and the polar solvent (III) are recirculated to the hydrogenation reactor.

**2.** The process according to claim 1, wherein a homogeneous catalyst comprising ruthenium is used.

**3.** The process according to claims 1 and 2, wherein a metal-organic complex comprising an element of group 8, 9 or 10 of the Periodic Table and at least one phosphine group having at least one unbranched or branched, acyclic or cyclic, aliphatic radical having from 1 to 12 carbon atoms, where individual carbon atoms can also be substituted by >P-, is used as homogeneous catalyst.

**4.** The process according to any of claims 1 to 3, wherein, in feature (e), the bottoms from the distillation unit are separated into a phase comprising the free tertiary amine (I) and a phase comprising the polar solvent and the two phases are recirculated separately to the hydrogenation reactor, with the phase comprising the free tertiary amine being recirculated to the hydrogenation reactor via an extraction unit and homogeneous catalyst being extracted from the separated off liquid phase (A) in said extraction unit before the formic acid/amine adduct (B) in the separated-off liquid phase (A) is thermally dissociated according to feature (c) into free formic acid and free tertiary amine (I) in a distillation unit.

**5.** The process according to any of claims 1 to 4, wherein an amine of the general formula (Ia)

$NR^1R^2R^3$ (Ia),

where the radicals $R^1$ to $R^3$ are identical or different and are each, independently of one another, an unbranched or branched, acyclic or cyclic, aliphatic, araliphatic or aromatic radical having in each case from 1 to 16 carbon atoms, where individual carbon atoms can also be substituted, independently of one another, by a hetero group selected from the group consisting of -0- and >N- or two or all three radicals can also be joined to one another to form a chain comprising at least four atoms in each case, is used as tertiary amine (I).

**6.** The process according to claim 5, wherein an amine of the general formula (Ia) in which the radicals $R^1$ to $R^3$ are selected independently from the group consisting of $C_1$-$C_{12}$-alkyl, $C_5$-$C_8$-cycloalkyl, benzyl and phenyl is used as tertiary amine (I).

**7.** The process according to claim 5 or 6, wherein a saturated amine of the general formula (Ia) is used as tertiary

amine (I).

**8.** The process according to claim 7, wherein an amine of the general formula (Ia) in which the radicals $R^1$ to $R^3$ are selected independently from the group consisting of $C_5$-$C_8$-alkyl is used as tertiary amine (I).

**9.** The process according to any of claims 1 to 8, wherein an aliphatic, saturated hydrocarbon having from 2 to 5 OH groups or a formic ester thereof is used as polar solvent (III).

**10.** The process according to any of claims 1 to 9, wherein the hydrogenation is carried out at a molar ratio of carbon dioxide to tertiary amine (I) of from 0.1 to 10.

**11.** The process according to any of claims 1 to 10, wherein the separation of the one liquid phase (A) enriched with the formic acid/amine adduct (II) and the polar solvent (III) from the other liquid phase (B) enriched with the tertiary amine (I) and the recirculation of the liquid phase (B) to the hydrogenation reactor are carried out at a pressure of from 1 to 30 MPa abs.

## Revendications

**1.** Procédé de préparation d'acide formique par hydrogénation de dioxyde de carbone en présence d'un catalyseur contenant un élément du groupe 8, 9 ou 10 du tableau périodique, d'une amine tertiaire (I) et d'un solvant polaire (III), à une pression de 0,2 à 30 MPa abs. et à une température de 20 à 200 °C, par formation de deux phases liquides, séparation de la phase liquide (A) enrichie avec le produit d'addition acide formique/amine (II) de l'autre phase liquide (B) et recyclage de la phase liquide (B) dans le réacteur d'hydrogénation, **caractérisé en ce qu'**un catalyseur homogène est utilisé en tant que catalyseur et celui-ci se présente enrichi dans la phase liquide (B) avec l'amine tertiaire (I) ;

(a) une amine qui présente à une pression de 1 013 hPa abs. un point d'ébullition supérieur d'au moins 5 °C à l'acide formique et qui se présente enrichie dans la phase liquide (B) étant utilisée en tant qu'amine tertiaire (I) ;
(b) un solvant dont le facteur électrostatique est $\geq 200 \cdot 10^{-30}$ Cm et qui présente à une pression de 1 013 hPa abs. un point d'ébullition supérieur d'au moins 5 °C à l'acide formique et qui se présente enrichi dans la phase liquide (A) étant utilisé en tant que solvant polaire (III) ;
(c) le produit d'addition acide formique/amine (II) de la phase liquide (A) séparée étant clivé thermiquement en acide formique libre et amine tertiaire (I) libre dans une unité de distillation ;
(d) l'acide formique libre étant séparé par distillation ; et
(e) l'amine tertiaire (I) libre contenue dans le fond de l'unité de distillation et le solvant polaire (III) étant recyclés dans le réacteur d'hydrogénation.

**2.** Procédé selon la revendication 1, **caractérisé en ce qu'**un catalyseur homogène contenant du ruthénium est utilisé.

**3.** Procédé selon les revendications 1 à 2, **caractérisé en ce qu'**un complexe métallo-organique qui contient un élément du groupe 8, 9 ou 10 du tableau périodique et au moins un groupe phosphine comprenant au moins un radical aliphatique, non ramifié ou ramifié, acyclique ou cyclique, de 1 à 12 atomes de carbone, des atomes de carbone individuels pouvant également être substitués par >P-, est utilisé en tant que catalyseur homogène.

**4.** Procédé selon les revendications 1 à 3, **caractérisé en ce que**, selon la caractéristique (e), le fond de l'unité de distillation est séparé en une phase contenant l'amine tertiaire (I) libre et une phase contenant le solvant polaire (III) et les deux phases sont recyclées séparément dans le réacteur d'hydrogénation, la phase contenant l'amine tertiaire (I) libre étant recyclée dans le réacteur d'hydrogénation par le biais d'une unité d'extraction et le catalyseur homogène étant extrait de la phase liquide (A) séparée dans l'unité d'extraction mentionnée avant que le produit d'addition acide formique/amine (II) de la phase liquide (A) séparée ne soit clivé thermiquement dans une unité de distillation selon la caractéristique (c) en acide formique libre et amine tertiaire (I) libre.

**5.** Procédé selon les revendications 1 à 4, **caractérisé en ce qu'**une amine de formule générale (Ia)

$NR^1R^2R^3$ (Ia)

dans laquelle les radicaux $R^1$ à $R^3$ sont identiques ou différents et représentent indépendamment les uns des autres

un radical non ramifié ou ramifié, acyclique ou cyclique, aliphatique, araliphatique ou aromatique, contenant à chaque fois 1 à 16 atomes de carbone, des atomes de carbone individuels pouvant également être substitués indépendamment les uns des autres par un hétérogroupe choisi dans le groupe constitué par -0- et >N-, et deux des radicaux ou les trois radicaux pouvant également être reliés les uns aux autres en formant une chaîne qui comprend à chaque fois au moins quatre atomes,
est utilisée en tant qu'amine tertiaire (I).

6. Procédé selon la revendication 5, **caractérisé en ce qu'**une amine de formule générale (Ia), dans laquelle les radicaux $R^1$ à $R^3$ sont choisis indépendamment les uns des autres dans le groupe constitué par alkyle en $C_1$ à $C_{12}$, cycloalkyle en $C_5$ à $C_8$, benzyle et phényle, est utilisée en tant qu'amine tertiaire (I).

7. Procédé selon les revendications 5 à 6, **caractérisé en ce qu'**une amine saturée de formule générale (Ia) est utilisée en tant qu'amine tertiaire (I).

8. Procédé selon la revendication 7, **caractérisé en ce qu'**une amine de formule générale (Ia), dans laquelle les radicaux $R^1$ à $R^3$ sont choisis indépendamment les uns des autres dans le groupe constitué par alkyle en $C_5$ à $C_8$, est utilisée en tant qu'amine tertiaire (I).

9. Procédé selon les revendications 1 à 8, **caractérisé en ce qu'**un hydrocarbure aliphatique saturé contenant 2 à 5 groupes OH, ainsi que son ester de l'acide formique, sont utilisés en tant que solvant polaire (III).

10. Procédé selon les revendications 1 à 9, **caractérisé en ce que** l'hydrogénation est réalisée à un rapport molaire entre le dioxyde de carbone et l'amine tertiaire (I) de 0,1 à 10.

11. Procédé selon les revendications 1 à 10, **caractérisé en ce que** la séparation de la phase liquide (A) enrichie avec le produit d'addition acide formique/amine (II) et le solvant polaire (III) de l'autre phase liquide (B) enrichie avec l'amine tertiaire (I), ainsi que le recyclage de la phase liquide (B) dans le réacteur d'hydrogénation, sont réalisés à une pression de 1 à 30 MPa abs.

Fig. 1

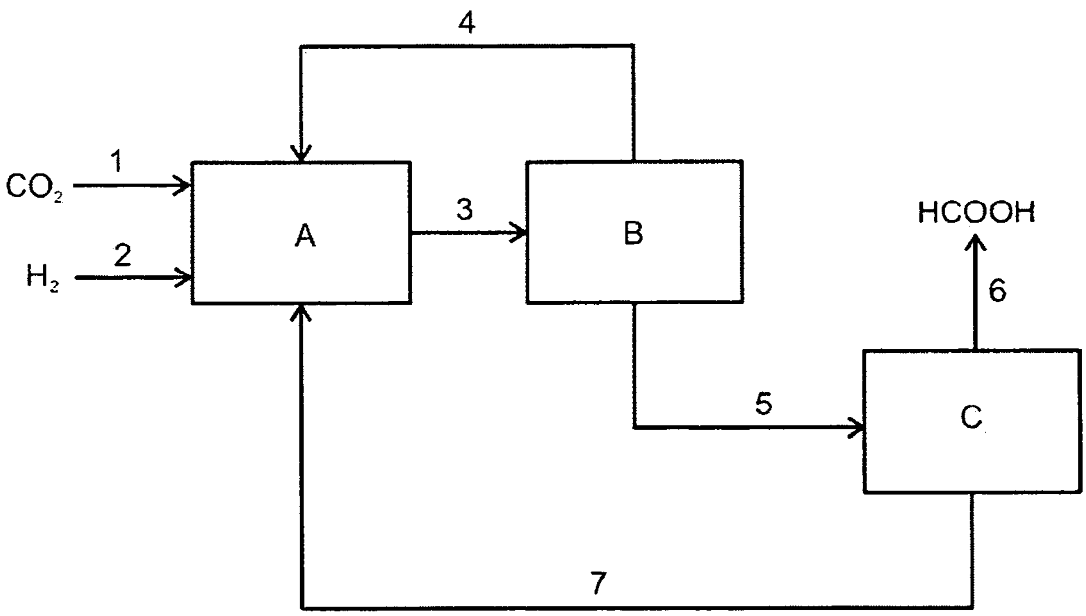

Fig. 2

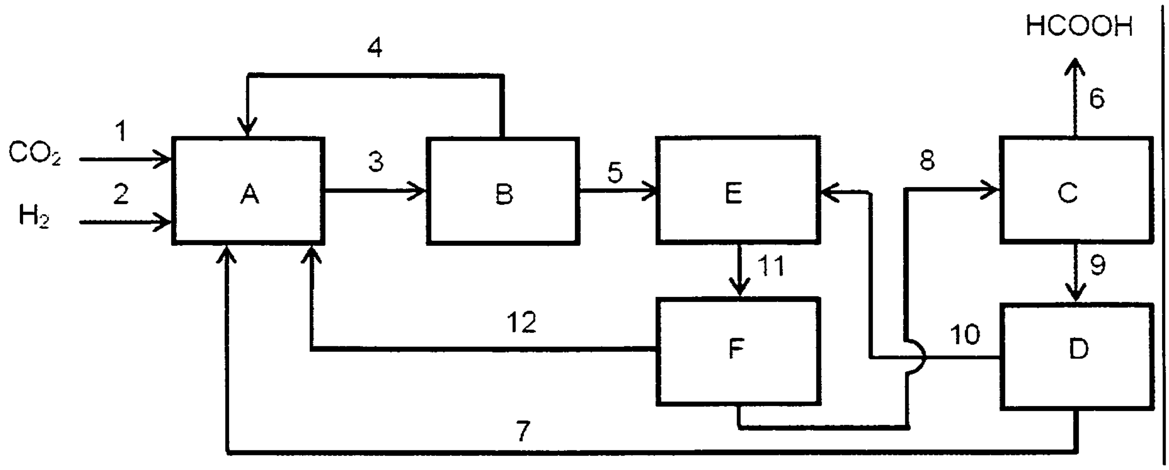

Fig. 3

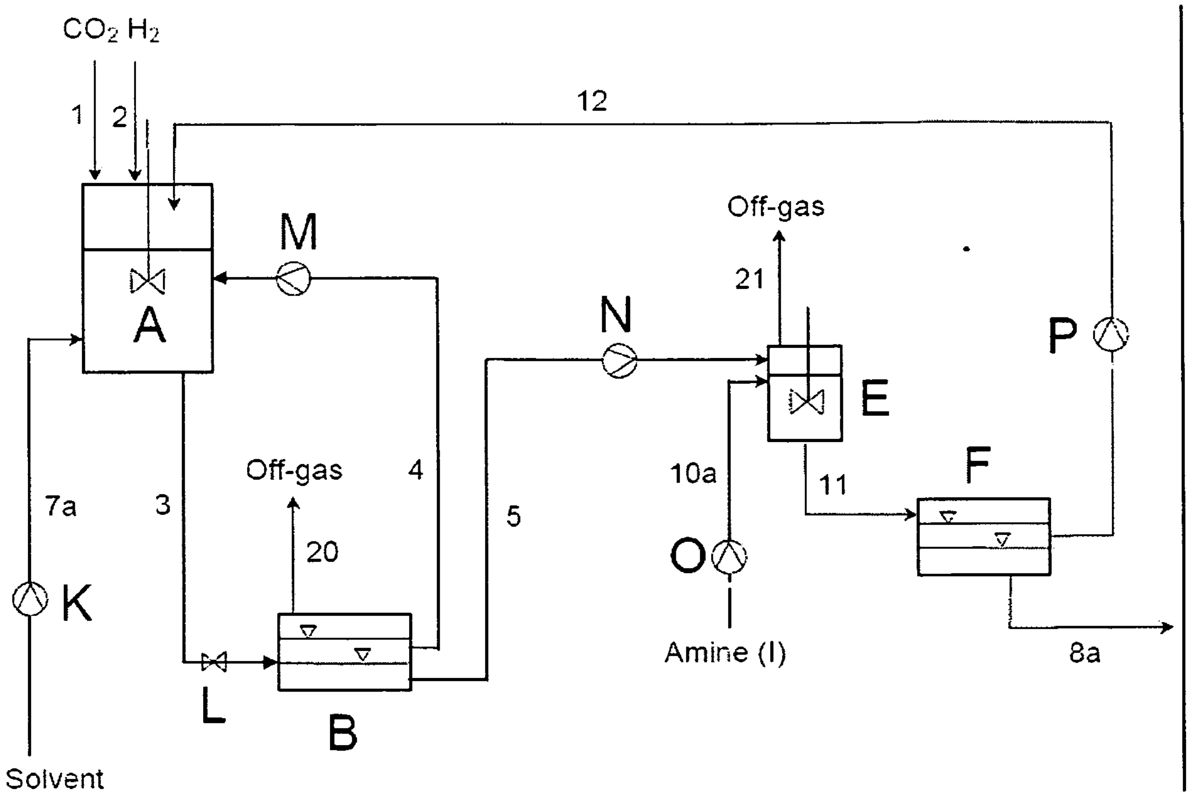
$CO_2$ $H_2$
1
2
12
M
A
Off-gas
21
N
P
E
F
7a
3
Off-gas
4
10a
11
5
20
O
K
Amine (I)
8a
L
B
Solvent

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008116799 A
- EP 0095321 A
- EP 0151510 A
- EP 0181078 A
- EP 0126524 A
- EP 0329337 A
- EP 0357243 A
- DE 4431233 A
- EP 0357234 A
- WO 2006021411 A
- DE 3428319 A
- EP 0563831 A


### In der Beschreibung aufgeführte Nicht-Patentliteratur


- **C.REICHARDT.** Solvents and Solvent Effects in Organic Chemistry. Wiley-VCH Verlag GmbH & Co KGaA, 2003
- Reactor Types and Their Industrial Applications. **K.D. HENKEL.** Ullmann's Encylcopedia of Industrial Chemistry. Wiley-VCH Verlag GmbH & Co. KGaA, 2005
- Liquid-Liquid Extraction. **E. MÜLLER et al.** Ullmann's Encylcopedia of Industrial Chemistry. Wiley-VCH Verlag GmbH & Co. KGaA, 2005